# EUROPEAN PATENT APPLICATION

(11) **EP 2 343 063 A1**
(43) Date of publication of application: **13.07.2011**
(21) Application number: 09180821.2
(22) Date of filing: 28.12.2009
(51) Int. Cl.: A61K 31/095, A61P 9/00, A61P 11/00, A61P 1/00

(54) **Pharmaceutical preparations comprising a COS releasing compound**

(71) Applicant: Universität Wien, 1010 Wien (AT); Ludwig Boltzmann Cluster für Kardiovaskuläre Forschung, 1090 Wien (AT)
(72) Inventor: Erker, Thomas, 1140, Wien (AT); Brunhofer, Gerda, 1140, Wien (AT); Studenik, Christian, 1070, Wien (AT); Podesser, Bruno, 1060, Wien (AT); Pomper, Gabriela, 1090, Wien (AT)
(74) Representative: Bühler, Dirk

(57) **Abstract**

The present invention relates to pharmaceutical compositions comprising a COS releasing compound and to methods of preparing such compositions. Said pharmaceutical compositions may preferably be used for the treatment and/or the prevention of cardiovascular diseases.

## Description

### FIELD OF THE INVENTION

The present invention relates to pharmaceutical compositions comprising a COS releasing compound and to methods of preparing the same. Said compositions seem to be potent vasodilators and may thus be used for the treatment and/or prevention of cardiovascular diseases, e.g. the treatment and/or prevention of a myocardial infarction.

### BACKGROUND OF THE INVENTION

Cardiovascular diseases accounted for 43% of all deaths in the year 2008. Therefore, the successful treatment of such diseases represents one of the major goals in the medical field.

The cardiovascular system comprises the heart, the blood and the blood vessels (arteries and veins). It is *inter alia* responsible for the transport of nutrients, electrolytes, gases (such as O₂), hormones and the like as well as for the homeostasis of the body by maintaining and stabilizing certain factors such as pH and temperature. The system is also implicated in the transport of cells, such as immune cells in order to fight diseases.

Several factors influence the cardiovascular system, among them vasoconstriction, vasodilation and the adhesion of cells to blood vessels (e.g. leukocyte adhesion to endothelial cells in case of inflammations).

Vasoconstriction is a process in order to narrow the blood vessels and results from the contraction of the muscular walls of the blood vessels, particularly arteries, arterioles and veins. As a result, the blood flow is restricted or decreased, leading e.g. to the retention of body heat and to a decreased transport of the above-mentioned substances. Furthermore, vasoconstriction may result in an increase of systemic blood pressure. However, vasoconstriction can also be localized to certain areas of the cardiovascular system resulting in specific effects in said areas only.

Vasodilation is the converse process, i.e. the widening of the blood vessels. As a result, the blood flow is increased; furthermore, the blood pressure decreases. As with vasoconstriction, the effects may be systemic or local.

Effects due to the process of vasoconstriction seem to be particularly implicated in a number of cardiovascular diseases.

Due to advanced research in the field of cardiovascular diseases during the last decades, several endogenous signalling molecules implicated in the regulation of the cardiovascular system could be identified.

Thus, nitric oxide (NO) and carbon monoxide (CO) were found to be important signalling molecules, which are produced endogenously. Furthermore, therapies based on the release of NO and CO display protective effects for the brain, the heart and circulation against a number of cardiovascular diseases. Recently, a third member of such endogenously produced signalling molecules implicated in the regulation of the cardiovascular system could be identified, namely hydrogen sulfide (H₂S). *Inter alia,* H₂S has been found to induce vasodilation and inhibit leukocyte-endothelial cell interactions in the circulation (see e.g. Lefer DJ, "A new gaseous signalling molecule emerges: Cardioprotective role of hydrogen sulfide", PNAS, 104, no. 46, Nov 13, 2007, pages 17907 - 17908).

Exogenous compounds influencing the levels of such signalling molecules thus represent attractive drug candidates. On the one hand, inhibitory compounds for the biosynthesis of the signalling molecules may result in decreased levels. On the other hand, compound acting as donor compounds for the signalling molecules may lead to increased levels. For H₂S, the effects of such H₂S inhibitors and H₂S donors in animal models of diseases are summarized e.g. in Szabo C, "Hydrogen sulphide and its therapeutic potential", Nat Rev Drug Disc, 6(11), Nov 2007, pages 917 - 935.

NO, CO as well as H₂S display vasodilatory effects and are thus *inter alia* responsible for the widening of blood vessels. Compounds acting e.g. as donors of NO, CO and H₂S may therefore be used as vasodilators and may thus be classified as cardioprotective substances.

However, there are still side effects associated with therapies based on substances influencing the endogenous signalling molecules as mentioned above and their levels, respectively, e.g. undesired effects on the heart (such as an impaired myocardial energy balance, distorted electrolyte levels or variations in the heart rate). Furthermore, there is a constant need for identifying novel endogenous signalling molecules implicated in the cardiovascular system since such novel mediators may e.g. be more specific compared to the above mentioned signalling molecules. This of course also includes the identification of exogenous compounds influencing the levels of such novel, endogenous signalling molecules. Compounds acting on said novel molecules may thus display fewer side effects and an increased efficacy profile.

Thus, there is the need for novel compounds, which act on the cardiovascular system and may be used in the treatment and/or prevention of cardiovascular diseases.

### OBJECTS AND SUMMARY OF THE INVENTION

It is one object of the present invention to provide a pharmaceutical composition comprising a COS releasing compound. In a preferred embodiment, said pharmaceutical composition is used for the treatment and/or prevention of a cardiovascular disease.

Another object of the present invention is to provide a method of preparing a pharmaceutical composition comprising a COS releasing compound.

It is also an object of the present invention to provide the use of COS or a COS releasing compound as a vasodilator.

These and other objects as they will become apparent from the ensuing description are attained by the subject matter of the independent claims. The dependent claims relate to some of the preferred embodiments of the present invention.

The inventors have surprisingly found that COS releasing compounds seem to be potent vasodilators, most likely due to the release of the signalling molecule COS, and that compositions comprising COS releasing compounds may thus be pharmaceutically used.

In a preferred embodiment of the first aspect, the present invention relates to a pharmaceutical composition comprising a COS releasing compound of formula (I): 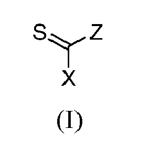 wherein
X is a substituted or unsubstituted heteroaryl or O-R₁;
wherein R₁ is selected from the group consisting of substituted or
unsubstituted C1-C10 alkyl, substituted or unsubstituted C3-C8 cycloalkyl, substituted or unsubstituted C2-C10 alkenyl, substituted or unsubstituted C4-C8 cycloalkenyl, -(C1-C10)(substituted or unsubstituted aryl) and substituted or unsubstituted aryl; Z is W-R₂ or NR₃R₄
wherein W is S or O;
wherein R₂ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl, substituted or unsubstituted heteroaryl, -(C1-C10)(substituted or unsubstituted aryl or heteroaryl), -(C1-C10)OH, -(C0-C10)C(O)R₅ -(C0-C10)C(O)OR₅, -(C1-C10)OC(O)R₅, -(C1-C10)OC(S)R₅ and -(C1-C10)OR₅;
wherein R₅ is selected from the group consisting of H, substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl and substituted or unsubstituted heteroaryl;
wherein R₃ and R₄ independently from each other are selected from the group consisting of H, substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl, substituted or unsubstituted heteroaryl, -(C1-C10)OH, -(CO-C10)C(O)R₅, -(C0-C10)C(O)OR₅, -(C1-C10)OC(O)R₅, -(C1-C10)OC(S)R₅, -(C1-C10)NHC(S)R₅, - (C1-C10)NHC(O)R₅ and -(C1-C10)OR₅; or
R₃ and R₄ are joined together to form a substituted or unsubstituted heteroaryl; or
NR₃R₄ form a structure of the following formula (II): 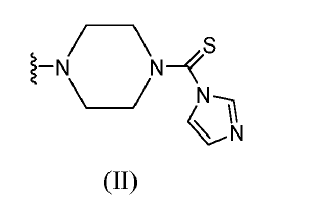 or a pharmaceutically acceptable salt thereof.

In certain preferred embodiment of the compounds according to formula (I), X is a substituted or unsubstituted heteroaryl.

In certain preferred embodiments of the compounds according to formula (I), X is a substituted or unsubstituted heteroaryl selected from the group consisting of imidazolyl, triazolyl and benzimidazolyl.

In certain preferred embodiments of the compounds according to formula (I), X is an unsubstituted heteroaryl, preferably selected from the group consisting of imidazolyl, triazolyl and benzimidazolyl.

In certain preferred embodiments of the compounds according to formula (I), X is an unsubstituted imidazolyl.

In certain preferred embodiments of the compounds according to formula (I), X is a substituted heteroaryl, preferably selected from the group consisting of imidazolyl, triazolyl and benzimidazolyl.

In certain preferred embodiments of the compounds according to formula (I), X is a substituted imidazolyl, preferably imidazolyl substituted with a C1-C6 alkyl, more preferably imidazolyl substituted with methyl. In said preferred embodiments it can furthermore be preferred that the imidazolyl is substituted with the C1-C6 alkyl, preferably methyl, at the following position:

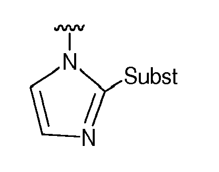

In certain preferred embodiments of the compounds according to formula (I), X is a substituted benzimidazolyl, preferably benzimidazolyl substituted with a C1-C6 alkyl, more preferably benzimidazolyl substituted with methyl. In said preferred embodiments it can furthermore be preferred that the benzimidazolyl is substituted with the C1-C6 alkyl, preferably methyl, at the following position:

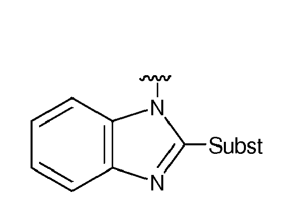

In certain preferred embodiments of the compounds according to formula (I), X is benzimidazolyl substituted with -O(C1-C6 alkyl), more preferably benzimidazolyl substituted with methoxy. In said preferred embodiments it can furthermore be preferred that the benzimidazolyl is substituted with -O(C1-C6 alkyl), preferably methoxy, at the following position:

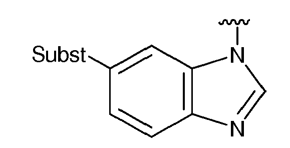

In certain preferred embodiments of the compounds according to formula (I), Z is W-R₂ with R₂ being an unsubstituted C1-C10 alkyl, preferably selected from the group consisting of methyl, ethyl, propyl, isopropyl and butyl.

In certain preferred embodiments of the compounds according to formula (I), Z is W-R₂ with R₂ being a substituted C1-C10 alkyl, preferably methyl being substituted with a -C6-C 14 aryl, more preferably methyl being substituted with phenyl.

In certain preferred embodiments of the compounds according to formula (I), Z is W-R₂ with R₂ being a substituted or unsubstituted aryl, preferably an unsubstituted aryl, more preferably phenyl.

In certain preferred embodiments of the compounds according to formula (I), Z is W-R₂ with R₂ being a substituted or unsubstituted C3-C8 cycloalkyl, preferably an unsubstituted C3-C8 cycloalkyl, more preferably cyclopentyl or cyclohexyl.

In certain preferred embodiments of the compounds according to formula (I), Z is W-R₂ with R₂ being -(C1-C10)OC(O)R₅, preferably -CH₂CH₂OC(O)R₅ or - CH₂OC(O)R₅, with R₅ being an unsubstituted C1-C10 alkyl, preferably methyl or isopropyl.

In certain preferred embodiments of the compounds according to formula (I), Z is W-R₂ with R₂ being -(C0-C10)C(O)OR₅, preferably -CH₂CH₂C(O)OR₅, with R₅ being an unsubstituted C1-C10 alkyl, preferably ethyl.

In certain preferred embodiments of the compounds according to formula (I), Z is W-R₂ with R₂ being -(C1-C10)(substituted or unsubstituted aryl), preferably - CH₂(substituted or unsubstituted aryl), more preferably -CH₂(substituted or unsubstituted phenyl). In embodiments, wherein R₂ is -CH₂(substituted phenyl), it can be preferred that the phenyl is substituted with one, two or three methoxy-groups.

In certain preferred embodiments of the compounds according to formula (I), Z is W-R₂ with R₂ being -(C1-C10)OC(S)R₅, preferably -CH₂CH₂OC(S)R₅, with R₅ being a heteroaryl, preferably imidazolyl.

In certain preferred embodiments of the compounds according to formula (I), Z is W-R₂ with W being S.

In certain preferred embodiments of the compounds according to formula (I), Z is NR₃R₄ with R₃ and R₄ joined together to form an unsubstituted heteroaryl, preferably imidazolyl.

In certain preferred embodiments of the compounds according to formula (I), Z is NR₃R₄ with both R₃ and R₄ being an unsubstituted C1-C10 alkyl, preferably selected from the group consisting of ethyl, propyl, butyl, isobutyl, hexyl and octyl.

In certain preferred embodiments of the compounds according to formula (I), Z is NR₃R₄ with both R₃ and R₄ being an unsubstituted C3-C8 cycloalkyl, preferably cyclopentyl.

In certain preferred embodiments of the compounds according to formula (I), Z is NR₃R₄ with both R₃ and R₄ being a substituted C1-C10 alkyl, preferably methyl substituted with a -C6-C 14 aryl, preferably phenyl.

In certain preferred embodiments of the compounds according to formula (I), Z is NR₃R₄ with R₃ being H and R₄ being -(C1-C10)NHC(S)R₅, preferably CH₂CH₂NHC (S)R₅, with R₅ being a heteroaryl, preferably imidazolyl.

It needs to be understood that all the above mentioned preferred embodiments of the compounds according to formula (I) relate to pharmaceutical compositions comprising compounds corresponding to said preferred embodiments.

In an especially preferred embodiment of the first aspect, the present invention relates to a pharmaceutical composition comprising a compound corresponding to another preferred embodiment of the compound of formula (I) wherein X is O-R₁ and Z is W-R₂ with W being O resulting in a compound of formula (III): 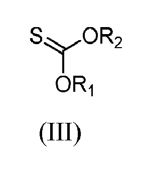 wherein R₁ and R₂ are as defined above, i.e.
wherein R₁ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, -(C1-C10)(substituted or unsubstituted aryl) and substituted or unsubstituted aryl;
wherein R₂ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl, substituted or unsubstituted heteroaryl, -(C1-C10)(substituted or unsubstituted aryl or heteroaryl), -(C1-C10)OH, -(C0-C10)C(O)R₅, -(C0-C10)C(O)OR₅, -(C1-C10)OC(O)R₅, -(C1-C10)OC(S)R₅ and -(C1-C10)OR₅;
wherein R₅ is selected from the group consisting of H, substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl and substituted or unsubstituted heteroaryl;
or a pharmaceutically acceptable salt thereof.

Thus, the compound of formula (III) is within the scope of a compound of formula (I), but represents a preferred subgroup of formula (I).

Within said subgroup of compounds according to formula (III), further preferred embodiments can be defined as outlined in the following.

In certain preferred embodiments of the compounds according to formula (III), R₁ is a substituted or unsubstituted C1-C10 alkyl, and/or R₂ is a substituted or unsubstituted C1-C10 alkyl.

It can furthermore be preferred for compounds according to formula (III) that both, R₁ and R₂ are a substituted C1-C10 alkyl, wherein both of said C1-C10 alkyls are preferably selected from methyl, ethyl or propyl, and both of said alkyls selected from methyl, ethyl or propyl are preferably substituted with -N(C1-C6 alkyl)(C1-C6 alkyl), more preferably substituted with -N(CH₃)(CH₃).

It can also be preferred that said substituted C1-C10 alkyl is the same for R₁ and R₂, e.g. R₁ is a substituted methyl and R₂ is a substituted methyl.

In certain other preferred embodiments of the compounds according to formula (III), R₁ is a substituted or unsubstituted aryl, and/or R₂ is a substituted or unsubstituted aryl.

In certain preferred embodiments of the compounds according to formula (III), R₁ is an unsubstituted aryl, preferably phenyl, and/or R₂ is an unsubstituted aryl, preferably phenyl.

It needs to be understood that all the above mentioned preferred embodiments of the compounds according to formula (III) again relate to pharmaceutical compositions comprising compounds corresponding to said preferred embodiments.

Furthermore, in a preferred embodiment of the first aspect of the invention, the hydrochloride (HCl) salt of the compound of formula (I) [and thus also the hydrochloride (HCl) salt of the compound of formula (III)] is comprised in the pharmaceutical composition.

In further preferred embodiments of the first aspect, the present invention relates to pharmaceutical compositions comprising a COS releasing compound as outlined above [including the preferred embodiments of said composition, wherein the above mentioned preferred compounds according to formulae (I) and (III) are comprised in the composition], wherein said compositions comprise said COS releasing compound as the only pharmaceutically active agent.

In an especially preferred embodiment of the present invention, the present invention relates to a pharmaceutical composition comprising a COS releasing compound as outlined above [including the preferred embodiments of said composition, wherein the above mentioned preferred compounds according to formulae (I) and (III) are comprised in the composition] for the use in the treatment and/or prevention of a cardiovascular disease.

The just mentioned preferred embodiment may also be formulated as a method of treating a cardiovascular disease in a human or animal being comprising administering to a subject, in an amount effective to treat said disease, a COS releasing compound, wherein said compound is preferably chosen from the above mentioned preferred compounds according to formulae (I) and (III).

In preferred embodiments of the use of a pharmaceutical composition comprising a COS releasing compound in the treatment and/or prevention of a cardiovascular disease, said disease is selected from the group of diseases comprising infarction, preferably myocardial infarction; ischemia- reperfusion injuries; protection of organs during surgery, preferably heart surgery or transplantation surgery; misregulated blood pressure, preferably increased blood pressure; circulatory disorders; cramps and motility-problems of the gastrointestinal tract; irritable bowel syndrome; cramps and dyskinesia of the biliary and urinary tract; cholecystopathia; dysmenorrhoidal problems; cramps at the female genital organs as well as spasms of the muscular soft tissues during delivery (tokolyse); pylorospasms of a baby; spasms during physicals, such as during gastroduodenal-endoscopy or during X-ray; arterial hypertension; pulmonary hypertension; therapy of the hypertension-crisis; coronary heart diseases, such as chronic stable angina pectoris or vasospastic angina; chronic insufficiency of the heart; arterial occlusions, such as Claudicatio intermittens; ischemic neurological deficiencies due to cerebral vasospasms following aneurysmatic subarachnoidal-bleeding; deficiencies of the brain during ageing; bronchospasms during asthma bronchiale; spastic bronchitis; Status Asthmaticus; Morbus Raynaud and vascular-caused cramps of the calves and the toes; and increased intraocular pressure (glaucoma).

In the second aspect mentioned above, the present invention relates to a method of preparing a pharmaceutical composition comprising a COS releasing compound comprising at least the steps of:
a) Providing a COS releasing compound;
b) Mixing said compound with at least one pharmaceutically acceptable
   excipient.

In a particularly preferred embodiment of the above mentioned method of the second aspect, said COS releasing compound is a compound of formula (I) 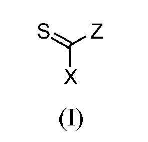 wherein
X is a substituted or unsubstituted heteroaryl or O-R₁;
wherein R₁ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, -(C1-C10)(substituted or unsubstituted aryl) and substituted or unsubstituted aryl;
Z is W-R₂ or NR₃R₄
wherein W is S or O;
wherein R₂ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl, substituted or unsubstituted heteroaryl, -(C1-C10)(substituted or unsubstituted aryl or heteroaryl), -(C1-C10)OH, -(C0-C10)C(O)R₅, -(C0-C10)C(O)OR₅, -(C1-C10)OC(O)R₅, -(C1-C10)OC(S)R₅ and -(C1-C10)OR₅;
wherein R₅ is selected from the group consisting of H, substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl and substituted or unsubstituted heteroaryl;
wherein R₃ and R₄ independently from each other are selected from the group consisting of H, substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl, substituted or unsubstituted heteroaryl, -(C1-C10)OH, -(C0-C10)C(O)R₅, -(C0-C10)C(O)OR₅, -(C1-C10)OC(O)R₅, -(C1-C10)OC(S)R₅, -(C1-C10)NHC(S)R₅, - (C1-C10)NHC(O)R₅ and -(C1-C10)OR₅; or
R₃ and R₄ are joined together to form a substituted or unsubstituted heteroaryl; or
NR₃R₄ form a structure of the following formula (II): 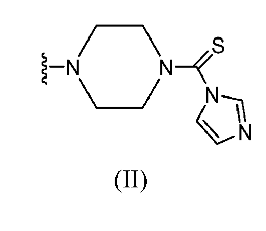 or a pharmaceutically acceptable salt thereof.

Furthermore, in other preferred embodiments of the second aspect as mentioned above, said COS releasing compound is a compound corresponding to the preferred embodiments of formula (I) as outlined above for the first aspect of the invention.

In an especially preferred embodiment of the second aspect of the invention, said COS releasing compound is a compound of formula (III), corresponding to a subgroup of formula (I) as defined above: 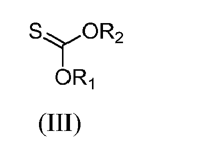 wherein R₁ and R₂ are as defined above, i.e.
wherein R₁ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, -(C1-C10)(substituted or unsubstituted aryl) and substituted or unsubstituted aryl;
wherein R₂ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl, substituted or unsubstituted heteroaryl, -(C1-C10)(substituted or unsubstituted aryl or heteroaryl), -(C1-C10)OH, -(C0-C10)C(O)R₅, -(C0-C10)C(O)OR₅, -(C1-C10)OC(O)R₅, -(C1-C10)OC(S)R₅ and -(C1-C10)OR₅;
wherein R₅ is selected from the group consisting of H, substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl and substituted or unsubstituted heteroaryl;
or a pharmaceutically acceptable salt thereof.

In further preferred embodiments of said second aspect, a COS releasing compound corresponding to the preferred compounds according to formulae (I) and (III) as mentioned in the first aspect are provided in step a).

Furthermore, in a preferred embodiment of the second aspect of the invention, said pharmaceutically acceptable excipient is preferably pharmaceutically inactive and selected from the group of excipients comprising coating materials, film-forming materials, fillers, disintegrating agents, release-modifying materials, carrier materials, diluents, binding agents and other adjuvants.

In an also preferred embodiment of the second aspect of the invention, the hydrochloride (HCl) salt of the compound of formula (I) [and thus also the hydrochloride (HCl) salt of the compound of formula (III)] is provided in step a).

In the third aspect, the present invention is concerned with the use of COS or a COS releasing compound as a vasodilator.

In a preferred embodiment of the third aspect, the present invention is concerned with the use of a COS releasing compound as mentioned above in the first aspect and preferred embodiments thereof [including compounds according to formula (III) and the preferred embodiments thereof] as a vasodilator.

### DESCRIPTION OF THE FIGURES

Fig. 1: Fig. 1 depicts the coronary flow ("CF", given in mvmin/g) over a time period of 60 minutes. During the first 15 minutes, there was baseline perfusion in both the control (n=8) and the test-population (n=8). Following base line perfusion, 10 µM CRC1HCl was administered in the test-population for 10 minutes. The coronary flow was monitored in both groups and is depicted together with the deviation at each time point within both groups (see Example 4.4 for details).
Fig. 2: Fig. 2 shows the heart rate ("HR", in bpm) over a time period of 60 minutes for the experiment described in Example 4.4 for both the control group and the test-population.
Fig. 3: Fig. 3 shows the aortic pressure ("AoP", in mmHg) over a time period of 60 minutes for the experiment described in Example 4.4 for both the control group and the test-population.

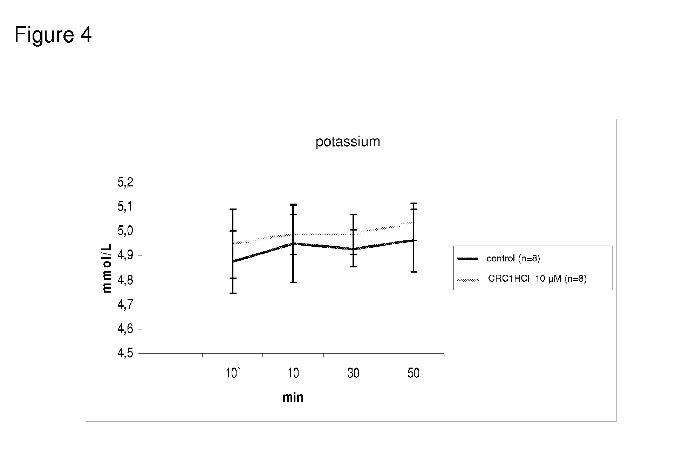

Fig. 4: Fig. 4 shows the potassium concentration in the coronary effusate (in mmol/l) with a baseline value at time point 10' and measurements 10, 30 and 50 minutes after administration of CRC1HCl for the experiment described in Example 4.4 for both the control group and the test-population.

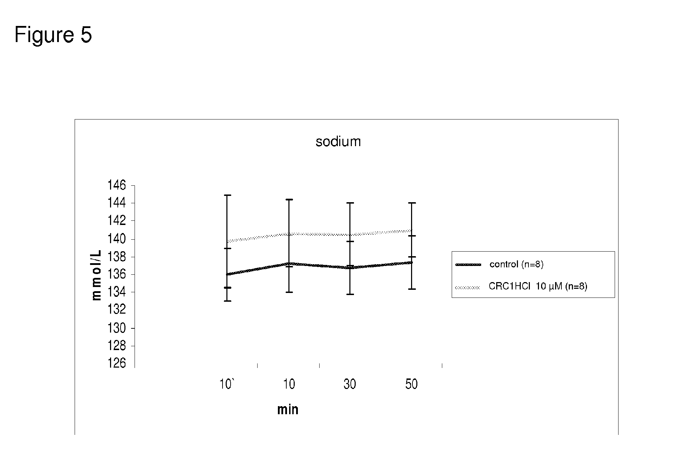

Fig. 5: Fig. 5 shows the sodium concentration in the coronary effusate (in mmol/l) with a baseline value at time point 10' and measurements 10, 30 and 50 minutes after administration of CRC1HCl for the experiment described in Example 4.4 for both the control group and the test-population.

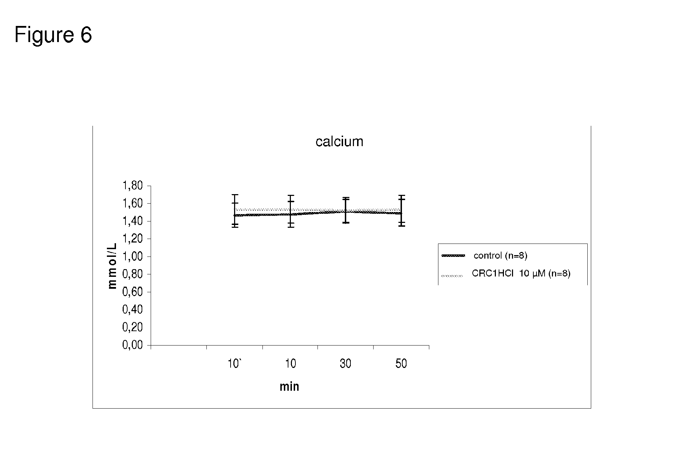

Fig. 6: Fig. 6 shows the calcium concentration in the coronary effusate (in mmol/l) with a baseline value at time point 10' and measurements 10, 30 and 50 minutes after administration of CRC1HCl for the experiment described in Example 4.4 for both the control group and the test-population.

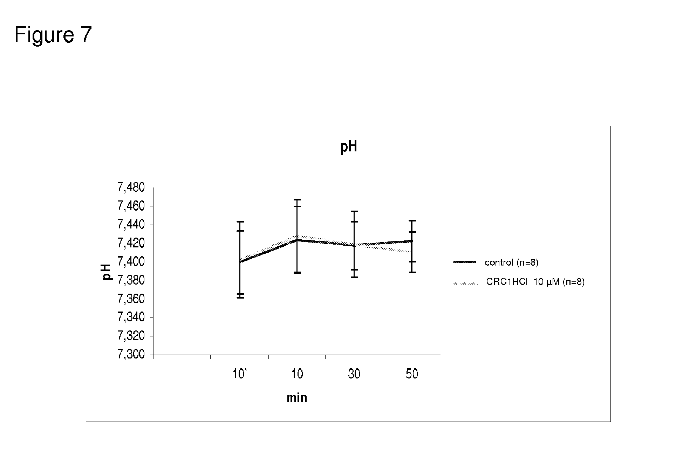

Fig. 7: Fig. 7 shows the pH of the coronary effusate (in a range of from 7.3 to 7.5) with a baseline value at time point 10' and measurements 10, 30 and 50 minutes after administration of CRC1HCl for the experiment described in Example 4.4 for both the control group and the test-population.

### DETAILED DESCRIPTION OF THE INVENTION

As already set out above, the present invention partially resides in the surprising finding that COS releasing compounds seem to be potent vasodilators, most likely due to the release of the signaling molecule COS. Thus, such compounds may be used in pharmaceutical compositions.

### 1. Definitions

Before some of the embodiments of the present invention are described in more detail, the following definitions are introduced.

As used in the specification and the claims, the singular forms of "a" and "an" also include the corresponding plurals unless the context clearly dictates otherwise.

The terms "about" and "approximately" in the context of the present invention denotes an interval of accuracy that a person skilled in the art will understand to still ensure the technical effect of the feature in question. The term typically indicates a deviation from the indicated numerical value of ±10% and preferably ±5%.

It needs to be understood that the term "comprising" is not limiting. For the purposes of the present invention, the term "consisting of" is considered to be a preferred embodiment of the term "comprising of". If hereinafter a group is defined to comprise at least a certain number of embodiments, this is also meant to encompass a group which preferably consists of these embodiments only.

The term "COS" or "carbonyl sulfide" as used herein corresponds to a compound of formula (IV):

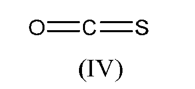

The term "COS releasing compound" as used herein defines a compound, from which COS is released during a hydrolysis reaction according to the following general scheme depicted for the compound of formula (I), wherein the substituents are as defined above:

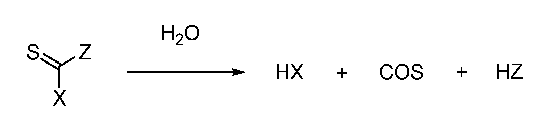

As set out above, X may be an unsubstituted heteroaryl such as benzimidazolyl, such that an exemplary hydrolysis reaction can be depicted as follows:

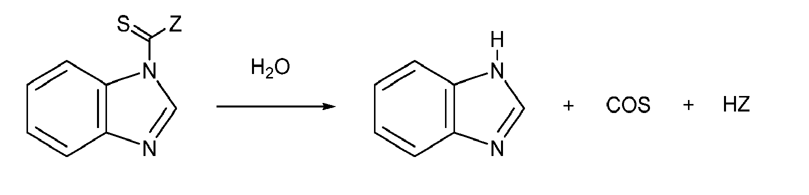

As also set out above, X may be O-R₁ and Z may be W-R₂ with W being O (corresponding to a compound of formula (III) with substituents R₁ and R₂ as defined above) such that an exemplary hydrolysis reaction can be depicted as follows:

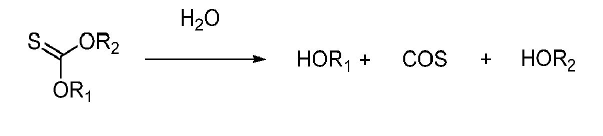

However, it is clear to the skilled person that COS may further be hydrolyzed to H₂S (such that the compound may also be described as "COS/H₂S-releasing compound", see below) according to the following reaction scheme:

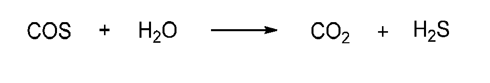

Therefore, it needs to be understood that the term "COS releasing compound" also comprises the definition for the compound as "H₂S releasing compound". The term "COS releasing compound" may thus also be understood as referring to a "COS/H₂S releasing compound". However, H₂S seems to be released in a smaller amount than COS and the observed biological effects of compounds according to the present invention seem to be due to COS rather than H₂S. However, H₂S released from a compound according to the present invention may also contribute to the observed effects.

Furthermore, the skilled person is aware that the just stated hydrolysis-reactions can indeed take place under physiological conditions in a body (due to e.g. the presence of H₂O in body fluids and at physiological pH).

In this regard, it should be noted that such reactions may also occur in a specific environment, e.g. in a certain region (such as a specific tissue) and/or a specific system (such as the cardiovascular system) only, due to the presence of endogenous factors, such as enzymes, influencing and/or catalyzing necessary steps prior to the hydrolysis reaction and/or the hydrolysis reaction itself such that the COS release is locally restricted to a certain area and/or system.

In general, the number of carbon atoms present in a given group is designated "Cx-Cy" where x and y are the lower and upper limits, respectively. For example, a group designated as "C1-C10" contains from 1 to 10 carbon atoms. The carbon number as used in the definitions herein refers to carbon backbone and carbon branching, but does not include carbon atoms of the substituents. General examples of alkyl groups include methyl, propyl, isopropyl, butyl, n-butyl, isobutyl, sec-butyl, tert-butyl, and pentyl.

"Carbon branching" or "branched alkyl" means that one or more alkyl groups such as methyl, ethyl or propyl, replace one or both hydrogens in a -CH₂- group of a linear alkyl chain.

The term "alkyl" refers to a hydrocarbon chain that may be a straight chain or branched chain, containing the indicated number of carbon atoms. For example, C1-C10 indicates that the group may have from 1 to 10 (inclusive) carbon atoms in it.

For example, the term "C1-C3 alkyl" refers to a straight or branched chain saturated hydrocarbon containing 1-3 carbon atoms. Examples of a C1-C3 alkyl group include, but are not limited to, methyl, ethyl, propyl and isopropyl.

For example, the term "C6-C10 alkyl" refers to a straight or branched chain saturated hydrocarbon containing 6-10 carbon atoms. Examples of a C6-C10 alkyl group include, but are not limited to, hexyl, octyl and decyl.

The term "cycloalkyl" refers to a saturated cycloalkyl group. The term "C3-C8 cycloalkyl", for example, refers to a non-aromatic mono- or multicyclic hydrocarbon ring system having a single radical and 3-8 carbon atoms. Exemplary monocyclic cycloalkyl rings include cyclopropyl, cyclopentyl, and cyclohexyl.

The term "alkenyl" refers to a straight or branched chain unsaturated hydrocarbon. The term "C2-C10 alkenyl" refers to a straight or branched chain unsaturated hydrocarbon containing 2-10 carbon atoms and at least one double bond. Examples of a C2-C10 alkenyl group include, but are not limited to, ethylene, propylene, 1-butylene, 2-butylene, isobutylene, sec-butylene, 1-pentene, 2-pentene, isopentene, 1-hexene, 2-hexene, 3-hexene, isohexene, 1-heptene, 2-heptene, 3-heptene, 1-octene, 2-octene, 3-octene, 4-octene, 1-nonene, 2-nonene, 3-nonene, 4-nonene, 1-decene, 2-decene, 3-decene, 4-decene and 5-decene.

The term "cycloalkenyl" refers to an unsaturated cycloalkyl group. The term "C4-C8 cycloalkenyl" means a non-aromatic monocyclic or multicyclic hydrocarbon ring system containing a carbon-carbon double bond having a single radical and 4 to 8 carbon atoms. Exemplary monocyclic cycloalkenyl rings include cyclopentenyl, cyclohexenyl or cycloheptenyl.

The term "aryl" refers to an aromatic hydrocarbon group. If not otherwise specified in this specification, the term aryl refers to a C6-C14 aryl group. Examples of a C6-C14 aryl group include, but are not limited to, phenyl, 1-naphthyl, 2-naphthyl, 3-biphen-1-yl, anthryl, tetrahydronaphthyl, fluorenyl, indanyl, biphenylenyl, and acenaphthenyl groups.

The term "heteroaryl" refers to mono, bicyclic, and tricyclic aromatic groups of 5 to 13 atoms containing at least one heteroatom and at least one aromatic ring, if not specified otherwise. Heteroatom as used in the term heteroaryl refers to oxygen, sulfur and nitrogen.

Examples of monocyclic heteroaryls include, but are not limited to pyrrolyl, oxazinyl, thiazinyl, pyridinyl, diazinyl, triazinyl, tetrazinyl, imidazolyl, tetrazolyl, isoxazolyl, furanyl, furazanyl, oxazolyl, thiazolyl, thiophenyl, pyrazolyl, triazolyl, and pyrimidinyl. Examples of bicyclic heteroaryls include but are not limited to, benzimidazolyl, indolyl, indolinyl, isoquinolinyl, quinolinyl, quinazolinyl, benzothiophenyl, benzodioxolyl, benzo[1,2,5]oxadiazolyl, purinyl, benzisoxazolyl, benzoxazolyl, benzthiazolyl, benzodiazolyl, benzotriazolyl, isoindolyl and indazolyl. Examples of tricyclic heteroaryls include but are not limited to, dibenzofuranyl, dibenzothiophenyl, phenanthridinyl, and benzoquinolinyl.

In a preferred embodiment of the present invention, the term "heteroaryl" refers to a heteroaryl selected from the group of heteroaryls consisting of pyrrolyl, oxazinyl, thiazinyl, pyridinyl, diazinyl, triazinyl, tetrazinyl, imidazolyl, tetrazolyl, isoxazolyl, furanyl, furazanyl, oxazolyl, thiazolyl, thiophenyl, pyrazolyl, triazolyl, pyrimidinyl, benzimidazolyl, indolyl, indolinyl, isoquinolinyl, quinolinyl, quinazolinyl, benzothiophenyl, benzodioxolyl, benzo[1,2,5]oxadiazolyl, purinyl, benzisoxazolyl, benzoxazolyl, benzthiazolyl, benzodiazolyl, benzotriazolyl, isoindolyl, indazolyl, dibenzofuranyl, dibenzothiophenyl, phenanthridinyl, and benzoquinolinyl.

The term "substituted" as used herein refers to substituted moieties (including substituted C1-C10 alkyl, substituted C3-C8 cycloalkyl, substituted C2-C10 alkenyl, substituted C4-C8 cycloalkenyl, substituted aryl and substituted heteroaryl) bearing one or more of the following groups or substituents: halogen, -C1-C6 alkyl, -C2-C6 alkenyl, -hydroxyl, -NH₂, -NH(C1-C6 alkyl), -N(C1-C6 alkyl)(C1-C6 alkyl), -N(C1-C3 alkyl)C(O)(C1-C6 alkyl), -NHC(O)(C1-C6 alkyl), -NHC(O)H, -C(O)NH₂, - C(O)NH(C1-C6 alkyl), -C(O)N(C1-C6 alkyl)(C1-C6 alkyl), -CN, CHN(C1-C6 alkyl), -O(C1-C6 alkyl) such as e.g. -OCH₃ corresponding to methoxy, -C(O)OH, -C(O)O(C1-C6 alkyl), -(C1-C6 alkyl)C(O)O(C1-C6 alkyl), -C(O)(C1-C 6alkyl), - C6-C14 aryl, -C5-C9 heteroaryl, -C3-C8 cycloalkyl, -haloalkyl, -aminoalkyl, - OC(O)(C1-C6 alkyl), -C1-C6 carboxyamidoalkyl and/or -NO₂.

The term "halogen" includes fluoride, bromide, chloride or iodide.

The term "halo" means -F, -Cl, -Br or -I. An exemplary haloalkyl includes trifluoromethyl.

The invention disclosed herein is meant to encompass all pharmaceutically acceptable salts of the disclosed compounds. The pharmaceutically acceptable salts include, but are not limited to, metal salts such as sodium salt, potassium salt, cesium salt and the like; alkaline earth metals such as calcium salt, magnesium salt and the like; organic amine salts such as triethylamine salt, pyridine salt, picoline salt, ethanolamine salt, triethanolamine salt, dicyclohexylamine salt, N,N'-dibenzylethylenediamine salt and the like; inorganic acid salts such as hydrochloride, hydrobromide, sulfate, phosphate and the like; organic acid salts such as formate, acetate, trifluoroacetate, maleate, fumarate, tartrate and the like; sulfonates such as methanesulfonate, benzenesulfonate, p-toluenesulfonate, and the like; amino acid salts such as arginate, asparginate, glutamate and the like.

Some of the compounds disclosed herein may contain one or more asymmetric centers and may thus lead to enantiomers, diastereomers, and other stereoisomeric forms.

The present invention is also meant to encompass all such possible forms as well as their racemic and resolved forms and mixtures thereof, unless specified otherwise. When the compounds described herein contain olefinic double bonds or other centers of geometric asymmetry, and unless specified otherwise, it is intended to include both E and Z geometric isomers. All tautomers are intended to be encompassed by the present invention as well.

As used herein, the term "stereoisomers" is a general term for all isomers of individual molecules that differ only in the orientation of their atoms in space. It includes enantiomers and isomers of compounds with more than one chiral center that are not mirror images of one another (diastereomers).

The term "chiral center" refers to a carbon atom to which four different groups are attached.

The term "enantiomer" or "enantiomeric" refers to a molecule that is nonsuperimposeable on its mirror image and hence optically active wherein the enantiomer rotates the plane of polarized light in one direction and its mirror image rotates the plane of polarized light in the opposite direction.

The term "racemic" refers to a mixture of equal parts of enantiomers and which is optically inactive.

The term "resolution" refers to the separation or concentration or depletion of one of the two enantiomeric forms of a molecule.

"Pharmaceutically active agent" as used herein means that a compound is potent of modulating a response in a human or animal being *in vivo.* When reference is made to a compound as "the only pharmaceutically active agent", this is meant to describe that the activity of a corresponding pharmaceutical composition is due to said active agent only.

The term "treatment of a cardiovascular disease" indicates that a cardiovascular diseases has been diagnosed and the symptoms associated therewith can be abolished or at least improved by administration of a pharmaceutical composition comprising a COS releasing compound.

The term "prevention of a cardiovascular disease" means that there is an increased risk in a patient of suffering from a cardiovascular disease (e.g. due to the presence of specific markers or risk factors), which can be attenuated by administration of a pharmaceutical composition comprising a COS releasing compound; thus, the administration of such a composition reduces the likelihood of development of these diseases or at least alleviates the extent and/or frequency to which these diseases develop.

When the term "disease" is used in the present invention, it preferably refers to a disease in a human or animal being. In this regard, it is obvious for the skilled person that specific diseases can e.g. be attributed to specific impaired signaling processes in the body (e.g. via signaling molecules). However, it is also obvious for the skilled person that due to the complexity of signaling processes in a body, such a process is almost never influenced by a singular pathway only, but rather several signaling networks seem to be involved. In the context of the present invention, this means that the diseases referred to herein seem to be influenced by COS-signaling, without however being exclusively influenced thereby.

The term "pharmaceutically acceptable excipient" as used herein refers to compounds commonly comprised in pharmaceutical compositions, which are known to the skilled person. Such compounds or excipients are exemplary listed under 3. below. In view of the definition "pharmaceutically active agent" as given above, a pharmaceutically acceptable excipient may thus be defined as being pharmaceutically inactive.

The term "vasodilator" as used herein refers to a compound inducing the widening of the blood vessels.

### 2. Methods for synthesizing compounds of the present invention

In the following paragraphs, general schemes for synthesizing the COS releasing compounds of formula (I) of the present invention are outlined. It is important to note that obvious alternative routes may also be used when making the compounds; thus, such standard alternatives routes known and applied by the person skilled in the art are also meant to be encompassed by said schemes.

Furthermore, it should be noted that synthesis reactions for individual compounds according to the present invention are outlined in the Example section below.

For all substituents of the compounds mentioned in the following (i.e. R₂, R₃ and R₄), reference is made to the objects and summary part of the description, wherein said substituents are defined. X' is halo, "RT" stands for room temperature, and "THF" is tetrahydrofuran.

In the following schemes, the substituted or unsubstituted heteroaryl as defined for X of the compound of formula (I) is always imidazolyl starting from imidazole. However, the skilled person is clearly aware that, when using a different heteroaryl (such as e.g. triazole or benzimidazole, substituted or unsubstituted), X can easily be such a different heteroaryl as well.

In the following, an exemplary scheme to synthesize compounds according to formula (I), wherein X is imidazolyl and Z is W-R₂ with W being S, is given:

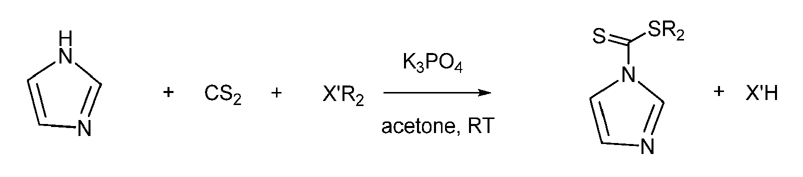

Alternatively, such compounds may be prepared according to the following scheme:

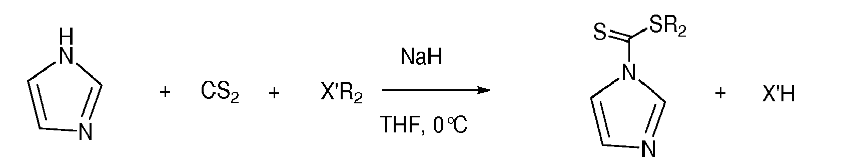

In the following, an exemplary scheme to synthesize compounds according to formula (I), wherein X is imidazolyl and Z is W-R₂ with W being O, is given:

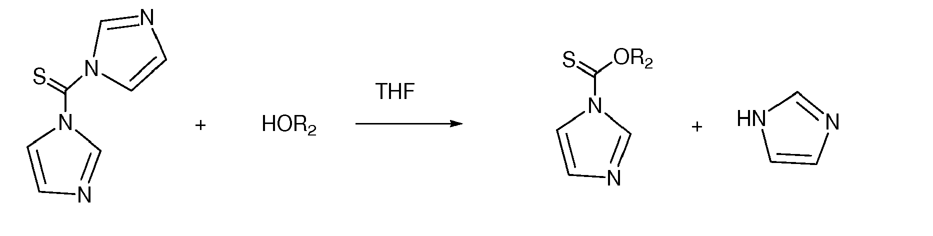

In the following, an exemplary scheme to synthesize compounds according to formula (I), wherein X is imidazolyl and Z is NR₃R₄ is given:

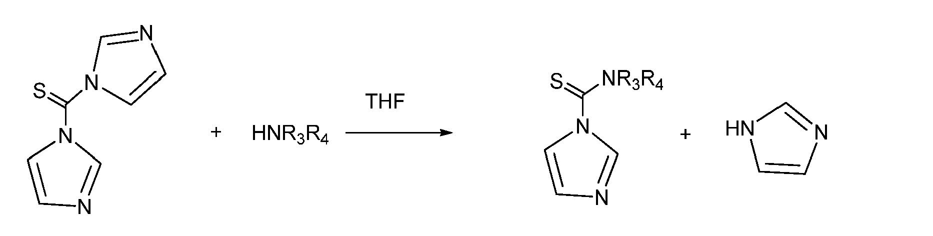

### 3. Pharmaceutical compositions

If a COS releasing compound (with COS releasing compounds as mentioned in the preferred embodiments of the first aspect [including compounds of formula (III)] above being particularly preferred) is comprised within a medicament, such a medicament may be formulated for oral, buccal, nasal, rectal, topical, transdermal or parenteral application. Parenteral application may include intravenous, intramuscular or subcutaneous administration. The COS releasing compound may be applied in pharmaceutically effective amounts, for example in the amounts as set out herein below.

The pharmaceutical compositions of the invention may be made from the compounds of the invention alone or they may comprise pharmaceutically acceptable excipients. Such compositions may also be designated as formulations.

As outlined above, the release of COS is most likely responsible for the observed effects. The predominant COS release should thus preferably take place at the desired area (e.g. a certain tissue) and/or system (e.g. the cardiovascular system) in need of COS in order to treat and/or prevent a disease (e.g. a cardiovascular disease). Accordingly, the pharmaceutical formulation should be chosen such that said local effect can be achieved.

The term "predominant" in this regard means that minor hydrolysis side reactions may take place prior to the compound having been delivered to the desired area and/or system. Most of the COS should, however, be released at the desired area and/or system.

Some of the COS releasing compounds according to formula (I) may, however, release COS in a spontaneous hydrolysis reaction due to the presence of H₂O in aqueous solutions, suspensions, and the like. Said compounds may also be referred to as being "instable compounds" in aqueous solutions. The determination of the stability of COS releasing compounds according to the present invention may easily be determined, e.g. by dissolving a compound to be tested in an aqueous solution and determining the amount of COS released over time.

In case of instable compounds, the skilled person is aware that some of the below mentioned pharmaceutical preparations (or formulations) can be preferred over others. The skilled person will thus try to avoid selecting a formulation for instable COS releasing compound comprising excipients, such as aqueous solutions, resulting in spontaneous hydrolysis reactions of the compound. Rather, the skilled person will preferably chose dry powder formulations and the like for such instable compounds.

Other COS releasing compounds according to the present invention seem to be stable in aqueous solutions and may thus be formulated in a way that seems to be most suitable to the skilled person in view of the administration route chosen and in view of patient compliance.

In this regard, the inventors have surprisingly found that a compound of formula (I) wherein X is O-R₁ and Z is W-R₂ with W being O, is stable in aqueous solutions, the compound corresponding to a compound of formula (III): 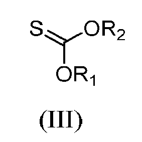 wherein R₁ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, -(C1-C10)(substituted or unsubstituted aryl) and substituted or unsubstituted aryl;
wherein R₂ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl, substituted or unsubstituted heteroaryl, -(C1-C10)(substituted or unsubstituted aryl or heteroaryl), -(C1-C10)OH, -(C0-C10)C(O)R₅, -(C0-C10)C(O)OR₅, -(C1-C10)OC(O)R₅, -(C1-C10)OC(S)R₅ and -(C1-C10)OR₅;
wherein R₅ is selected from the group consisting of H, substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl and substituted or unsubstituted heteroaryl;
or a pharmaceutically acceptable salt thereof.

In order to achieve the effect of COS release at the desired area and/or system for such stable compounds, endogenous factors at said area and/or system seem to be responsible for the COS release upon administration of the compound, e.g. enzymes catalyzing the corresponding hydrolysis reaction. Suitable endogenous factors seem to be *inter alia* present in the cardiovascular system and the compounds thus seem to be particularly effective when administered in order to treat and/or prevent a cardiovascular disease. Particularly important endogenous factors in this regard seem to be esterases, which are present in the blood serum resulting in a COS release at the desired area and system, respectively.

Therefore, a COS releasing compound of formula (III) may be particularly preferred in view of the stability and the localized release of COS at the desired area and/or system.

Pharmaceutical preparations comprising a COS releasing compound of formula (III) may thus be formulated in suitable formulations (e.g. in view of costs, administration routes, patient compliance and overall acceptance), such as oral dosage forms, e.g. tablets and the like as set out below.

Pharmaceutical dosage forms may be solid or liquid dosage forms or may have an intermediate, e.g. gel-like character depending *inter alia* on the route of administration.

In general, the inventive dosage forms will comprise various pharmaceutically acceptable excipients which will be selected depending on which functionality is to be achieved for the dosage form.

A "pharmaceutically acceptable excipient" in the meaning of the present invention can be any substance used for the preparation of pharmaceutical dosage forms, including but not limited to coating materials, film-forming materials, fillers, disintegrating agents, release-modifying materials, carrier materials, diluents, binding agents and other adjuvants. The term "carrier" denotes an organic or inorganic ingredient, natural or synthetic, with which the active ingredient is combined to facilitate the application. The components of the pharmaceutical compositions also are capable of being commingled with the compounds of the present invention, and with each other, in a manner such that there is no interaction which would substantially impair the desired pharmaceutical efficiency.

Typical pharmaceutically acceptable excipients include substances like sucrose, mannitol, sorbitol, starch and starch derivatives, lactose, and lubricating agents such as magnesium stearate, disintegrants and buffering agents.

In case that liquid dosage forms are considered for the present invention, these can include pharmaceutically acceptable emulsions, solutions, suspensions and syrups containing inert diluents commonly used in the art such as water. These dosage forms may contain e.g. microcrystalline cellulose for imparting bulk, alginic acid or sodium alginate as a suspending agent, methylcellulose as a viscosity enhancer and sweeteners/flavouring agents.

Further conventional excipients, which can be used in the aforementioned dosage forms depending on the functionality that is to be achieved for the dosage form, include pharmaceutically acceptable organic or inorganic carrier substances which do not react with the active compound. Suitable pharmaceutically acceptable carriers include, for instance, water, salt solutions, alcohols, oils, preferably vegetable oils, polyethylene glycols, gelatin, lactose, amylose, magnesium stearate, surfactants, perfume oil, fatty acid monoglycerides and diglycerides, petroethral fatty acid esters, hydroxymethyl-cellulose, polyvinylpyrrolidone and the like. The pharmaceutical preparations can be sterilized and if desired, mixed with auxiliary agents, like lubricants, preservatives, stabilizers, wetting agents, emulsifiers, salts for influencing osmotic pressure, buffers, colorings, flavoring and/or aromatic substances and the like which do not deleteriously react with the active compounds. For parenteral application, particularly suitable vehicles consist of solutions, preferably oily or aqueous solutions, as well as suspensions, emulsions, or implants.

The person skilled in the art is aware that the bioavailability of the COS releasing compound can be enhanced by micronisation of the formulations and the active agents using conventional techniques such as grinding, milling and spray-drying in the presence of suitable excipients or agents such as phospholipids or surfactants.

Injectable preparations of a COS releasing compound, for example sterile injectable aqueous or oleaginous suspensions, can be formulated according to the known art using suitable dispersing agents, wetting agents and/or suspending agents. A sterile injectable preparation can also be a sterile injectable solution or suspension in a nontoxic parenterally acceptable diluant or solvent. Among the acceptable vehicles and solvents that can be used are water and isotonic sodium chloride solution. Sterile oils are also conventionally used as solvent or suspending medium.

Suppositories for rectal administration of a COS releasing compound can be prepared by e.g. mixing the compound with a suitable non-irritating excipient such as cocoa butter, synthetic triglycerides and polyethylene glycols which are solid at room temperature but liquid at rectal temperature such that they will melt in the rectum and release the COS releasing compound from said suppositories.

For administration by inhalation, the compounds for use according to the present invention may be conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g. gelatin for use in an inhaler or insufflator may be formulated containing a powder mix of the compound and a suitable powder base such as lactose or starch.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Oral dosage forms may be a particularly preferred embodiment in view of patients' overall acceptance of this type of dosage forms. Oral dosage forms may be liquid or solid. Solid oral dosage forms can include e.g. tablets, troches, pills, capsules, powders, effervescent formulations, dragees and granules.

Pharmaceutical preparations for oral use can be obtained as solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate. Optionally the oral formulations may also be formulated in saline or buffers for neutralizing internal acid conditions or may be administered without any carriers.

According to another aspect of the present invention, the solid dosage form comprises a film coating. For example, the inventive dosage form may be in the form of a so-called film tablet. According to some aspects, the inventive dosage may comprise two or more film coating layers. The corresponding dosage form may be a bilayer or multilayer tablet.

As outlined above, suitable dosage forms according to the present invention may be in the form of a tablet, a dragee or a capsule. The capsule may be a two-piece hard gelatin capsule, a two-piece hydroxypropylmethylcellulose capsule, a two-piece capsule made of vegetable or plant-based cellulose or a two-piece capsule made of polysaccharide. The tablet may be a compressed tablet and/or a film coated tablet. The oral dosage forms may be formulated to ensure an immediate release of the COS releasing compound or a sustained release of the COS releasing compound.

According to another aspect of the present invention, the dosage form contains the COS releasing compound compressed together with an excipient. It may be especially preferred to prepare the inventive dosage from the COS releasing compound with a polymer. In this context, suitable polymers according to the present invention may be selected form the group comprising alginic acid, carboxymethylcellulose calcium, carboxymethylcellulose sodium, colloidal silicon dioxide, guar gum, magnesium aluminum silicate, methylcellulose, microcrystalline cellulose, cellulose, pregelatinized starch, sodium alginate, starch, ethylcellulose, gelatin, hydroxyethyl cellulose, hydroxypropyl cellulose, hydroxypropylmethyl cellulose, polymethacrylate, povidone, shellac and zein.

As set out above, the medicament may be formulated for topical application. Suitable pharmaceutical application forms for such an application may be a topical nasal spray, sublingual administration forms and controlled and/or sustained release skin patches.

For buccal administration, the compositions may take the form of tablets or lozenges formulated in conventional manner.

The pharmaceutical compositions in accordance with the present invention may not only comprise the COS releasing compound but also another pharmaceutically active agent. In case they do comprise (a) further active agent(s), said agent(s) is/are preferably one or more pharmaceutically active agents which are known to have a positive effect on the treatment and/or prevention of cardiovascular diseases, when administered to a patient in need of treatment thereof.

The compositions may conveniently be presented in unit dosage forms and may be prepared by any of the methods well known in the art of pharmacy. The methods can include the step of bringing the compounds into association with a carrier which constitutes one or more accessory ingredients. In general, the compositions are prepared by uniformly and intimately bringing the compounds into association with a liquid carrier, a finely divided solid carrier, or both, and then, if necessary, shaping the product. Liquid dose units are vials or ampoules. Solid dose units are tablets, capsules and suppositories.

As regards human patients, the COS releasing compound may be administered to a patient in an amount of about 25 mg to about 5000 mg, preferably of about 70 mg to about 500 mg per day.

A pharmaceutical composition comprising a COS releasing compound may in particular be used for the treatment of a cardiovascular disease selected from the group of diseases comprising infarction, preferably myocardial infarction; ischemia-reperfusion injuries; protection of organs during surgery, preferably heart surgery or a transplantation surgery; misregulated blood pressure, preferably increased blood pressure; circulatory disorders; cramps and motility-problems of the gastrointestinal tract; irritable bowel syndrome; cramps and dyskinesia of the biliary and urinary tract; cholecystopathia; dysmenorrhoidal problems; cramps at the female genital organs as well as spasms of the muscular soft tissues during delivery (tokolyse); pylorospasms of a baby; spasms during physicals, such as during gastroduodenal-endoscopy or during X-ray; arterial hypertension; pulmonary hypertension; therapy of the hypertension-crisis; coronary heart diseases, such as chronic stable angina pectoris or vasospastic angina; chronic insufficiency of the heart; arterial occlusions, such as Claudicatio intermittens; ischemic neurological deficiencies due to cerebral vasospasms following aneurysmatic subarachnoidal-bleeding; deficiencies of the brain during ageing; bronchospasms during asthma bronchiale; spastic bronchitis; Status Asthmaticus; Morbus Raynaud and vascular-caused cramps of the calves and the toes; and increased intraocular pressure (glaucoma).

### 4. Examples

In the following, examples of embodiments of the present invention are outlined. However, said examples should not be construed as limiting the scope of the present invention.

### 4.1. Synthesis of exemplary COS-releasing compounds according to the invention

The COS-releasing compound CRC1HCl was synthesized according to the following protocol: To a solution of 5 mmol (0.61 ml) 1-(methyldithiocarbonyl)imidazole in 5 ml anhydrous diethylether, 5 ml of HCl dissolved in diethylether (1.0 M) were slowly added. The immediately formed solid was filtered off and recrystallized in dichloromethane. The reaction yielded 95% (0.93 mg) of the desired product CRC1HCl as yellow crystals (mp 108-114°C).¹H-NMR (D₂O, 200 MHz): δ9.59-9.40 (m, 1H), 8.20-8.02 (m, 1H), 7.54-7.49 (m, 1H), 2.76 (s, 3H). ¹³C-NMR (D₂O, 50 MHz): δ197.7, 121.2, 119.9,21.1.

The COS-releasing compounds CRC13, CRC6 and CRC14 were synthesized according to the following general procedure: Potassium phosphate was added equimolar to a solution of 5 mmol heterocycle derivative in acetone. After 15 minutes an excess of CS₂, and after another 15 minutes the corresponding alkylhalogenide were added and stirred at room temperature. After completed reaction the solid was filtered off and the organic solvent was removed *in vacuo.* The so-obtained crude product was purified by column chromatography and recrystallisation.

CRC13 was synthesized by reaction of 5 mmol (0.34 g) imidazole and 5 mmol (0.61 g) 2-chloroethylacetate. After 24 hours the reaction was completed. Purification by column chromatography (ethylacetate) yielded 19% (0.22 g) of the desired compound as yellow oil.

In order to synthesize CRC13HCl, 1 ml of HCl dissolved in diethylether (1.0 M) was slowly added to a solution of 0.8 mmol (0.18 g) CRC13 in 3 ml anhydrous diethylether. The immediately formed solid was filtered off and recrystallized in dichloromethane. The reaction yielded 15% (0.03 g) of the desired product CRC13HCl as yellow crystals (mp 70-87°C). ¹H-NMR (D₂O, 200 MHz): δ9.62-9.50 (m, 1H), 8.23-8.14 (m, 1H), 7.58-7.50 (m, 1H), 4.40 (t, *J*=5.9 Hz, 2H), 3.77 (t, *J*=5.9 Hz, 2H), 2.01 (s, 3H). ¹³C-NMR (D₂O, 50 MHz): δ196.7, 174.9, 135.3, 122.2, 120.7, 62.0, 37.5, 21.4.

CRC6 was synthesized by reaction of 5 mmol (0.74 g) 5-methoxybenzimidazole and 5 mmol (0.71 g) methyliodide. After 3 hours the reaction was completed. Purification by column chromatography (ethylacetate) yielded 33% (0.39 g) of the desired compound CRC6 as yellow crystals (mp 64-87°C, isomeric forms). ¹H-NMR (CDCl₃, 200 MHz, isomeric forms): δ8.83 (s, 1H),8.75 (s, 1H), 8.48-8.37 (m, 1H), 8.22-8.12 (m, 1H), 7.73-7.60 (m, 1H), 7.31-7.22 (m, 1H), 7.08-6.93 (m, 2H), 3.88 (s, 3H), 3.88 (s, 3H), 2.83 (s, 3H), 2.83 (s, 3H). ¹³C-NMR (CDCl₃, 50 MHz, isomeric forms): δ198.9, 198.4, 158.5, 157.5, 146.4, 141.8, 140.3, 139.3, 127.2, 121.2, 116.2, 114.3, 113.5, 103.6, 100.2, 55.9, 55.7, 19.8, 19.8. MS m/z: 238 (M⁺, 27%), 91 (100%).

CRC14 was synthesized by reaction of 5 mmol (0.35 g) 1,2,4-triazole and 5 mmol (0.61 g) 2-chloroethylacetate. After 8 hours the reaction was completed. Purification by column chromatography (toluene/ethylacetate 6+4) yielded 17% (0.20 g) of the desired compound as yellow oil. ¹H-NMR (CDCl₃, 200 MHz): δ9.17 (s, 1H), 8.10 (s, 1H), 4.41 (t, *J*=6.2 Hz, 2H), 3.64 (t, *J*=6.2 Hz, 2H), 2.09 (s, 3H). ¹³C-NMR (CDCl₃, 50 MHz): δ197.4, 170.6, 153.4 (2H), 60.7, 34.7, 20.7. MS m/z: 231 (M⁺, 1 %), 43 (100%).

The COS-releasing compound CRC33 was synthesized according to the following protocol: A suspension of 5 mmol (0.12 g) sodium hydride in THF was added to a solution of 5 mmol (0.34 g) imidazole in THF. After 15 minutes an excess of CS₂ was added at 0°C. 60 minutes later 5 mmol (1.31 g) 3,4,5-trimethoxybenzylbromide were added at room temperature. After 22 hours the reaction was completed. The resulting solid was filtered off and the organic solvent was removed *in vacuo.* The so-obtained crude product was purified by column chromatography (toluene/ethylacetate 6+4) and recrystallized in petrolether. The reaction yielded 39% (0.63 g) of the desired compound CRC33 as yellow crystals (mp 110-113°C). ¹H-NMR (CDCl₃, 200 MHz): δ8.49 (s, 1H), 7.80-7.75 (m, 1H), 7.12-7.09 (m, 1H), 6.61 (s, 2H), 4.56 (s, 2H), 3.87 (s, 6H), 3.85 (s, 3H). ¹³C-NMR (CDCl₃, 50 MHz): δ197.3, 153.4 (2C), 137.9, 131.6 (2C), 128.8, 117.6, 106.4 (2C), 60.8, 56.1, 42.3. MS m/z: 324 (M⁺, 2%), 181 (100%).

The COS-releasing compounds CRC40 and CRC58 were synthesized by the following general procedure: To a solution of 5 mmol (0.89 g) thiocarbonyldiimidazole in 5 ml anhydrous THF, 4.5 mmol of the corresponding alcohol/amine derivative were added. After completed reaction the organic solvent was removed *in vacuo* and the crude product was purified by column chromatography.

CRC40 was synthesized by reaction of 4.5 mmol (0.33 g) 1-butanole. After 3 hours the reaction was completed. Purification by column chromatography (toluene/ethylacetate 2+8) yielded 94% (0.78 g) of the desired compound CRC40 as yellow oil.

To a solution of 3 mmol (0.55 g) CRC40 in 5 ml anhydrous diethylether, 3.2 ml of HCl dissolved in diethylether (1.0 M) were slowly added. The immediately formed solid was filtered off and the reaction yielded 95% (0.63 g) of the desired product CRC40HCl as white crystals (mp 74-77°C). ¹H-NMR (CDCl₃, 200 MHz): δ12.08 (s, broad, 1H), 9.59 (s, 1H), 7.94 (s, 1H), 7.57 (s, 1H), 4.79 (t, *J*=6.7 Hz, 2H), 2.20-1.92 (m, 2H), 1.69-1.35 (m, 2H), 1.02 (t, *J*=7.3 Hz, 3H). ¹³C-NMR (CDCl₃, 50 MHz): δ 179.5, 133.9, 121.3, 119.2, 77.0, 29.7, 19.0, 13.5.

CRC58 was synthesized by reaction of 4.5 mmol (0.83 g) dihexylamine. After 1.5 hours the reaction was completed. Purification by column chromatography (toluene/ethylacetate 2+8) yielded 93% (1.24 g) of the desired compound CRC58 as yellow oil. ¹H-NMR (CDCl₃, 200 MHz): δ7.77 (s, 1H), 7.15 (s, 1H), 7.08 (s, 1H), 4.08-3.25 (m, 4H), 1.82-1.58 (m, 4H), 1.42-1.18 (m, 12H), 1.00-0.78 (m, 6H). ¹³C-NMR (CDCl₃, 50 MHz): δ178.8, 136.7, 129.6, 118.9, 53.3 (2C), 31.2 (2C), 26.4 (2C), 22.4 (2C), 13.9 (2C). MS m/z: 295 (M⁺, 23%), 43 (100%).

The COS-releasing compound CRC66 was synthesized according to the following protocol: To a solution of 9.5 mmol (1.69 g) thiocarbonyldiimidazole in anhydrous THF, 4.5 mmol (0.40 g) of piperazine were added. After 18 hours the reaction was completed and the formed solid was filtered off and recrystallized in ethanol. The reaction yielded 48% (0.66 g) of the desired compound CRC66 as yellow crystals (mp 225-226°C). ¹H-NMR (*d*₆-DMSO, 200 MHz): δ8.07 (s, 1H), 7.65-7.45 (m, 1H), 7.18-6.95 (m, 1H), 4.78-3.50 (m, 8H). ¹³C-NMR (*d*₆-DMSO, 50 MHz): δ177.6, 137.6, 129.1, 119.9, 49.4 (4C). MS m/z: 306 (M⁺, 19%), 86 (100%).

The COS-releasing compound CRC72HCl was synthesized according to the following protocol: To a solution of 5 mmol (0.89 g) thiocarbonyldiimidazole in 5 ml anhydrous THF 15 mmol (1.34g) of N,N-dimethylethanolamine were added. After 4 hours the reaction was completed.

The organic solvent was removed *in vacuo* and the crude product was purified by column chromatography (toluene/ethylacetate/triethylamine 6+4+2). The reaction yielded 93% (0.92 g) of the desired compound CRC72 as yellow oil. To a solution of 3.5 mmol (0.74 g) CRC72 in 5 ml anhydrous diethylether 4 ml of HCl dissolved in diethylether (1.0 M) were slowly added. The immediately formed solid was filtered off. The reaction yielded 91 % (0.69 g) of the desired product (mp 135-137°C). ¹H-NMR (*d*₆-DMSO, 200 MHz): δ11.19 (s, 2H), 4.81 (s, 4H), 3.51 (s, 4H), 2.78-2.77 (m, 12H). ¹³C-NMR (*d*₆-DMSO, 50 MHz): δ193.3 (1C), 67.3 (2C), 54.1 (2C), 42.4 (4C).

### 4.2. Table of COS-releasing compounds

The following COS-releasing compounds were prepared according to the general reaction schemes given above and the detailed protocols given in 4.1., respectively:

| | |
|---|---|
| CRC1 | 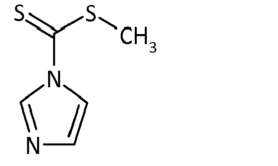 |
| CRC1HCl | 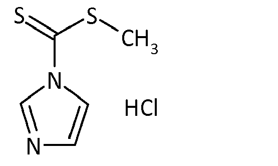 |
| CRC2 | 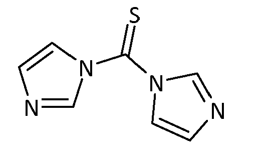 |
| CRC13 | 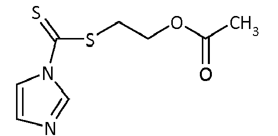 |
| CRC13HCl | 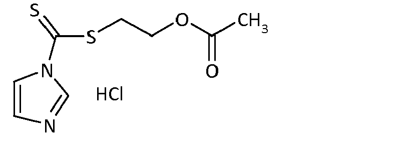 |
| CRC11 | 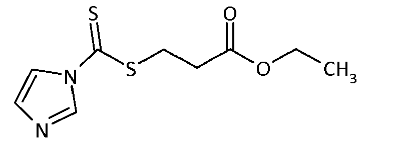 |
| CRC11HCl | 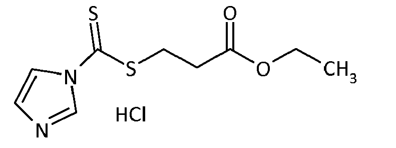 |
| CRC7 | 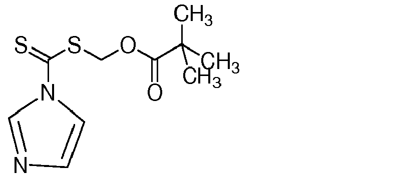 |
| CRC8 | 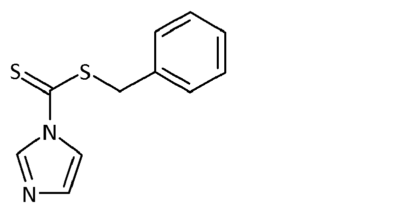 |
| CRC8HCl | 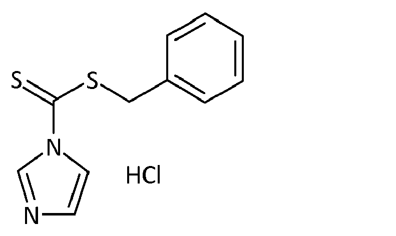 |
| CRC10 | 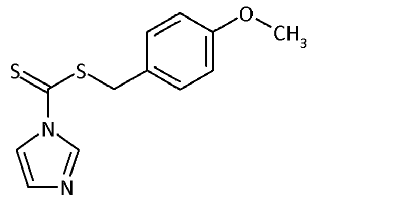 |
| CRC10HCl | 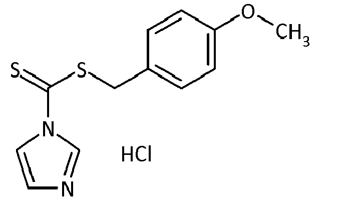 |
| CRC36 | 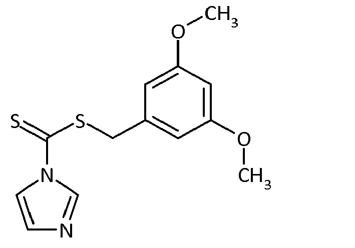 |
| CRC33 | 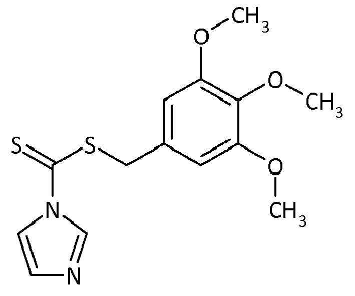 |
| CRC5 | 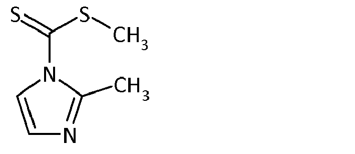 |
| CRC5HCl | 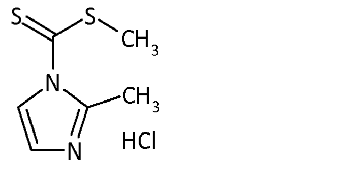 |
| CRC9 | 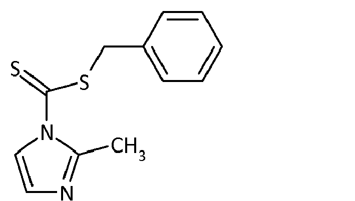 |
| CRC9HCl | 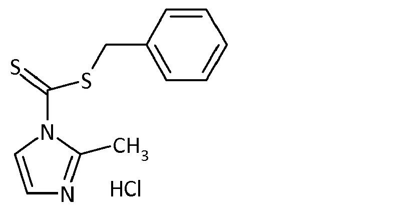 |
| CRC4 | 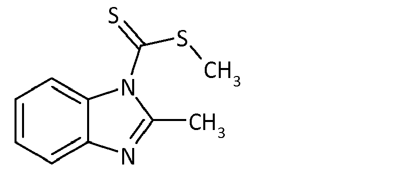 |
| CRC4HCl | 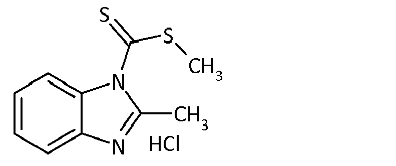 |
| CRC20 | 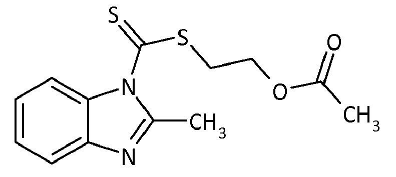 |
| CRC6 | 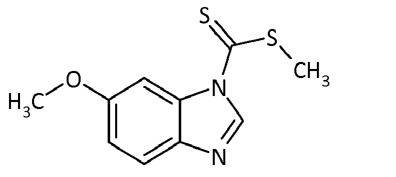 |
| CRC12 | 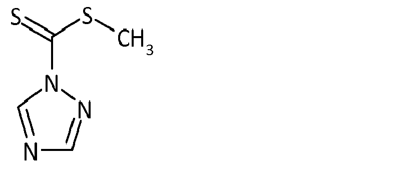 |
| CRC14 | 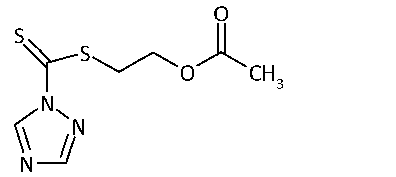 |
| CRC37 | 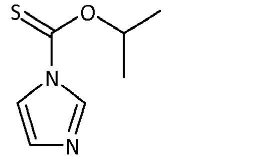 |
| CRC37HCl | 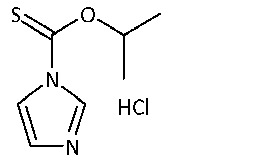 |
| CRC38 | 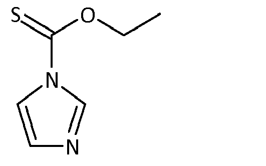 |
| CRC38HCl | 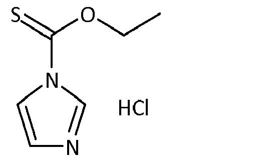 |
| CRC40 | 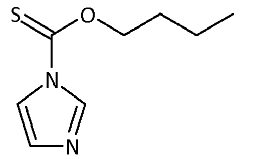 |
| CRC40HCl | 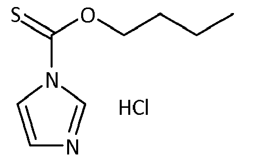 |
| CRC41 | 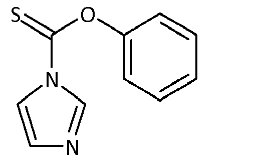 |
| CRC41HCl | 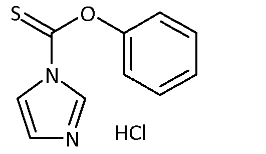 |
| CRC47 | 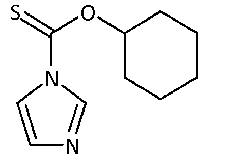 |
| CRC47HCl | 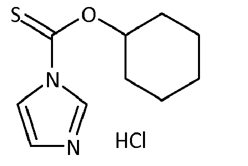 |
| CRC48 | 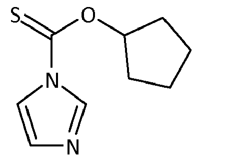 |
| CRC48HCl | 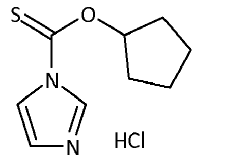 |
| CRC52 | 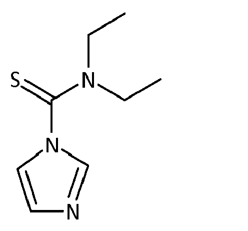 |
| CRC52HCl | 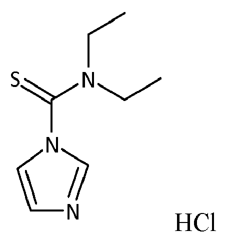 |
| CRC53 | 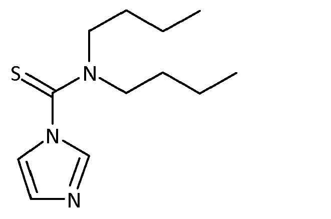 |
| CRC56 | 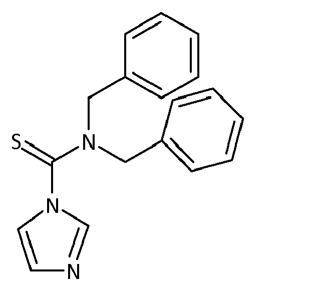 |
| CRC57 | 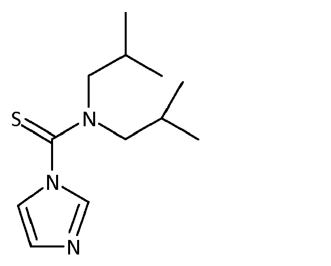 |
| CRC57HCl | 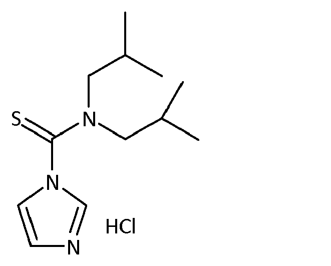 |
| CRC58 | 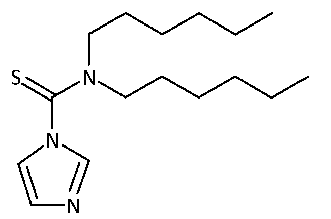 |
| CRC62 | 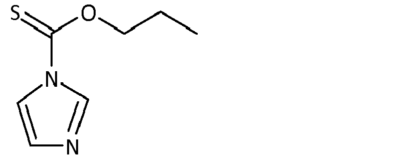 |
| CRC62HCl | 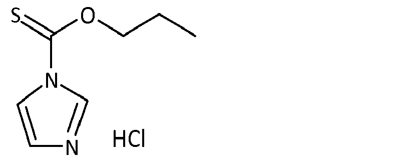 |
| CRC63 | 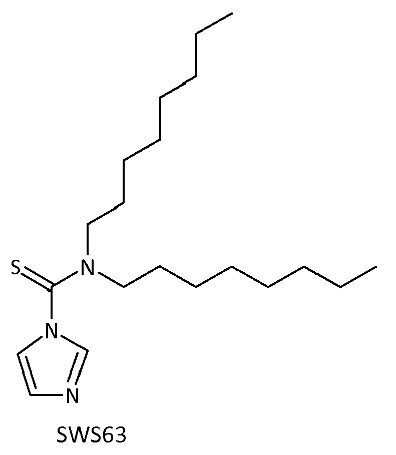 |
| CRC64 | 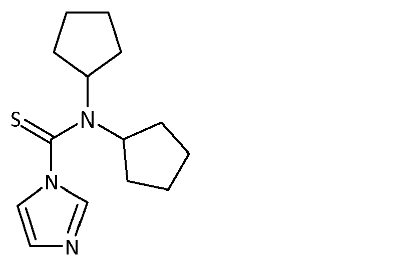 |
| CRC65 | 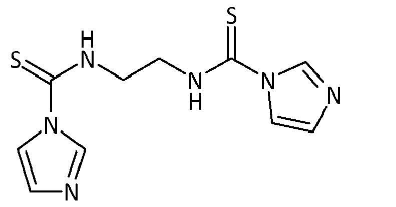 |
| CRC66 | 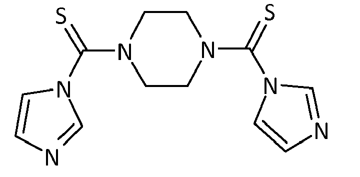 |
| CRC67 | 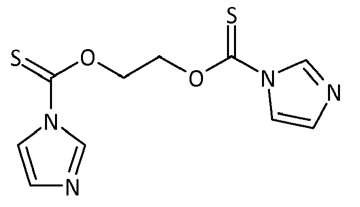 |
| CRC72 | 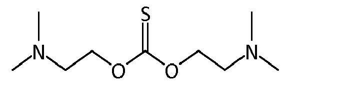 |
| CRC72HCl | 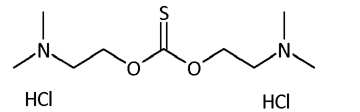 |

### 4.3. Activity of COS-releasing compounds on smooth muscle preparations

The COS-releasing compounds according to the present invention were tested on isolated muscle-preparations of the aorta, arteria pulmonalis, terminal ileum, papillary muscle and the right atrium (derived from guinea pigs) for their vasodilatory effect.

Guinea pigs of both sexes (340-480 g) were killed with a blow on the neck. After excision of the heart, the aorta and the ileum were dissected. The aorta was cleaned of loosely adhering fat and connective tissue and cut into rings of 2 mm width. The terminal portion of the ileum was removed and the 10 cm nearest to the caecum were discarded. The intestine was placed in a nutrient solution. The intestine was cleaned by flushing with nutrient solution and cut into pieces of 2 to 3 cm length. The arteria pulmonalis was dissected close to the heart, cleaned and cut into rings of 2 mm width. Papillary muscles were dissected from the right ventricle for contractility measurements. Purkinje fibres were carefully removed to prevent spontaneous activity. Only muscles with a diameter of less than 0.87 mm were used in order to have a sufficient oxygen supply. Chronotropic activity was tested on guinea-pig isolated right atria.

After isolation the preparations (aortic rings, terminal ilea, arteria pulmonalis rings, papillary muscles and right atria) were stored at room temperature in gassed (95% 02- 5% CO2) Krebs-Henseleit solution with the following composition (in mmol/l): NaCl 114.9, KCl 4.73, CaCl2 3.2, MgSO4 1.18, NaHCO3, 24.9, KH2PO4 1.18, glucose 10; pH 7.2-7.4.

Isometric contraction force of electrically stimulated papillary muscles (1 Hz), terminal ilea precontracted by 60 mmol/l KCl, aortic and arteria pulmonalis rings precontracted by 90 mmol/l KCl as well as spontaneous activity of right atria was measured by the method described by Reiter (Reiter, M. Bioassay of inotropic agents on the isolated papillary muscle. Arzneim. Forsch. 1967, 17, 1249-1253*).*

The preparations were placed in a continuously oxygenated (95% O2 and 5% CO2) bath of 35 ml nutrient solution to guarantee sufficient oxygen supply and appropriate pH as well as circulation of nutrient solution with and without test compound. The experiments were performed at a temperature of 37±1°C. The smooth and heart muscle preparations were connected with one end to a tissue holder and the other to a force transducer (TransbridgeTM, 4-Channel Transducer Amplifier, World Precision Instruments, Sarasota, FL, USA). Papillary muscles were electrically driven with an Anapulse Stimulator Model 301-T and an Isolation Unit Model 305-1 (WPI, Hamden, CT, USA) at a frequency of 1 Hz and a pulse duration of 3 ms. Amplitude of stimulation pulse was adjusted 10% above threshold level. Resting tension of either 3.92 mN (papillary muscles), 10.37 mN (right atria) or 19.6 mN (aortic and arteria pulmonalis rings) was kept constant throughout the experiments.

Signals were recorded with a chart recorder (BD 112 Dual Channel, Kipp & Zonen) and evaluated. For statistical analyses the arithmetic means and standard error of the mean (SEM) of n experiments were calculated. Statistical significance of the results was evaluated by the Student's t-test for paired observations. EC50-values were graphically determined from the concentration response curves.

Stock solutions of the compounds were dissolved in distilled water every day and were further diluted with modified Krebs-Henseleit solution to the required concentrations (1, 3, 10, 30 and 100 µmol/l).

The following EC₅₀-values were determined:

| **compound** | **Aorta** | **Arteria pulmonalis** | **Terminal Ileum** | **Papillary muscle** | **Right atrium** |
|---|---|---|---|---|---|
| **CRC1HCl** | **7,8 µM** | **6,9 µM** | **5,7 µM** | **78 µM** | **> 100 µM** |
| **CRC5HCl** | **6,3 µM** | **11,2 µM** | **9,5 µM** | **> 100 µM** | **> 100 µM** |
| **CRC11HCl** | **16 µM** | **19,8 µM** | **32,15 µM** | **> 100 µM** | **> 100 µM** |
| **CRC33** | **2,98 µM** | **7,9 µM** | **15,3 µM** | **> 100 µM** | **26,5 µM** |
| **CRC4HCl** | **22,78 µM** | **25,06 µM** | **25,59 µM** | **> 100 µM** | **55,97 µM** |
| **CRC8HCl** | **20,1 µM** | **63 µM** | **15,3 µM** | **26 µM** | **> 100 µM** |
| **CRC9HCl** | **3,36 µM** | **10,5 µM** | **17,15 µM** | **> 100 µM** | **15,3 µM** |
| **CRC10HCl** | **12,8 µM** | **24 µM** | **> 100 µM** | **> 100 µM** | **> 100 µM** |
| **CRC13HCl** | **5,75 µM** | **24 µM** | **> 100 µM** | **77 µM** | **> 100 µM** |

H₂S was used as reference compound. An EC₅₀ of 107 µM for H₂S was determined for the aorta-preparation.

Thus, the tested compounds (CRC1HCl, CRC5HCl, CRC11HCl, CRC33, CRC4HCl, CRC8HCl, CRC9HCl, CRC10HCl, CRC13HCl) display significantly lower EC₅₀-values compared to H₂S and therefore seem to be very potent vasodilators.

Furthermore, said compounds show a much weaker effect on the papillary muscle and almost no effect on the right atrium.

Compounds CRC59, CRC59HCl, CRC60 and CRC60HCl as indicated in the table below were used as negative controls in the present experimental setup. Due to the exchange of sulfur against oxygen, said compounds are not able to release COS. As expected, the compounds did not show vasodilatory activity [EC₅₀-values > 100 µM for CRC59HCl and CRC60HCl].

| | |
|---|---|
| CRC59 | 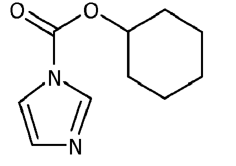 |
| CRC59HCl | 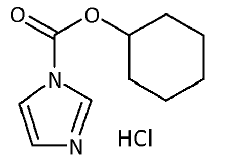 |
| CRC60 | 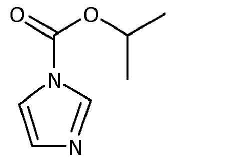 |
| CRC60HCl | 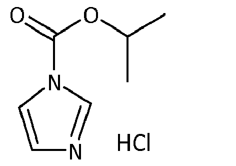 |

CRC72HCl as exemplary compound of a compound of formula (III) [corresponding to a subgroup of formula (I), see above] was also tested for a potential activity on smooth muscle preparations and found to be inactive in this experimental setup. This, however, does not indicate that CRC72HCl is generally inactive but rather shows that an aqueous solution (see protocol above) comprising such a compound is inactive. This indicates that CRC72HCl is stable in aqueous solutions and does not release COS under conditions, the experiments of 4.3 were performed.

### 4.4. Activity of COS-releasing compounds on the isolated mouse heart

Hearts from female OF-1 mice were excised, cannulated and perfused retrogradely, according to the Langendorff technique, with modified oxygenated Krebs-Henseleit-buffer at constant pressure. After 15 min of baseline perfusion, CRC1HCl (10 µM) was administered into the coronary perfusion system for 10 min. Coronary flow (CF), heart rate (HR), aortic pressure (AoP), concentrations of K⁺, Na⁺, Ca²⁺ and the pH-value were monitored throughout the whole experiment (CRC1HCl-group, n=8; control group, n=8).

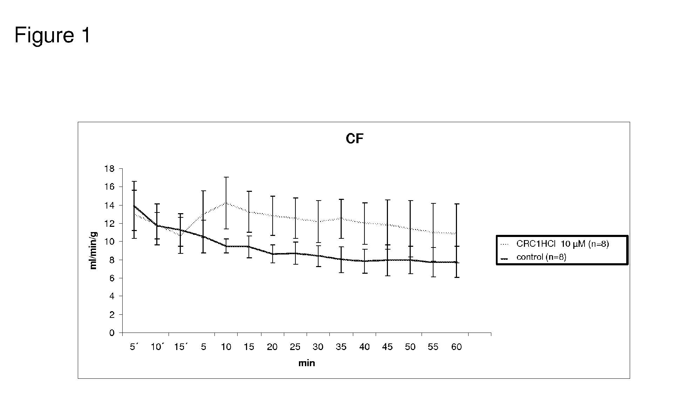

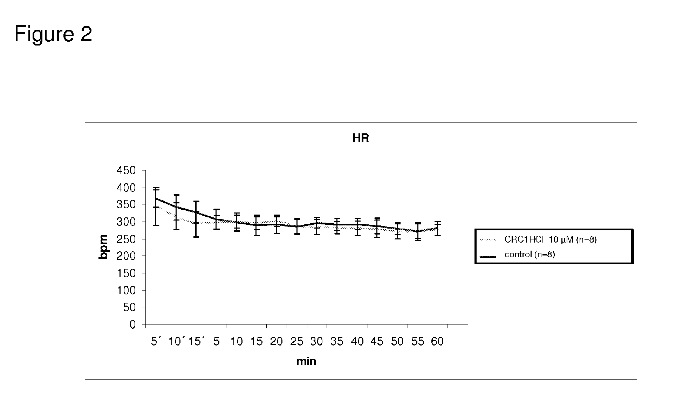

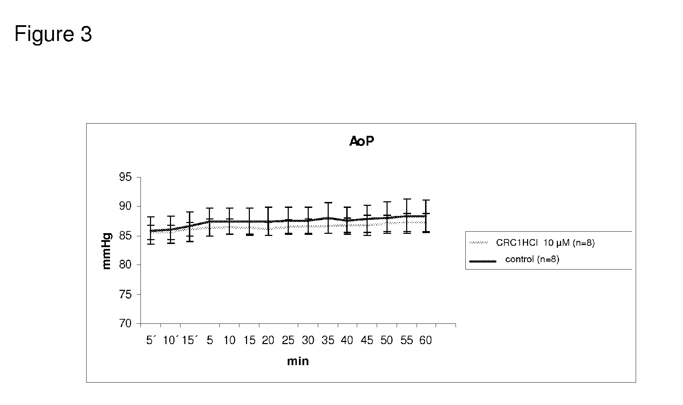

The results are depicted in figures 1 (CF), 2 (HR), 3 (AoP), 4 (K⁺), 5 (Na⁺), 6 (Ca²⁺) and 7 (pH).

In a second set of experiments in the setup as described above, nitro-L-arginine (L-NA) (n=6) and PNU-37883A (n=4) were evaluated as potential inhibitors of CRC1HCl (10 µM) activity. The compounds were added to final concentrations of 100 µM (L-NA) and 10 µM (PNU-37883A).

Also, the myocardial consumption of lactate and glucose was monitored in both the control-group and the CRC1HCl-group.

Clearly, the coronary flow was not altered during baseline perfusion (first 15 minutes). However, the coronary flow significantly increased during and 30 minutes after the administration of CRC1HCl compared to the control group (p<0.05). Furthermore, the heart rate, the aortic pressure, the concentrations of K⁺, Na⁺, Ca²⁺-ions and the pH-value were not significantly affected or altered, when CRC1HCl was administered. This also applied to the consumption of lactate and glucose (data not shown). Finally, the inhibition of ATP-sensitive potassium-channels (by PNU-37883A) and the inhibition of nitric oxide synthase (by nitro-L-arginine) had no effect on the vasodilatory effect of CRC1HCl (data not shown).

In summary, CRC1HCl thus significantly increases the coronary flow without affecting the myocardial energy balance, electrolyte levels and heart rate.

### 4.5. COS is released from a compound according to the present invention

In order to explain the significantly lower EC₅₀-values compared to H₂S, CRC1HCl as exemplary compound of the compounds of the present invention was tested for the release of the potential novel signalling molecule COS.

1 mmol CRC1HCl was dissolved in 1 ml water and kept at room temperature for 20 minutes. Then, a sample of the headspace was taken and injected into the GC-MS apparatus. The received MS spectrum showed peaks at m/z 34 and m/z 60 which indicated that H₂S as well as COS were present in the headspace of the dissolved compound.

It was found that COS is indeed released from CRC1HCl. Thus, the observed vasodilatory effects seem to be mediated via COS.

### 5. Particularly preferred embodiments of the invention

Particularly preferred embodiments of the invention relate to:
1. A pharmaceutical composition comprising a COS releasing compound.
2. Pharmaceutical composition according to 1, wherein said COS releasing compound is a compound of formula (I): 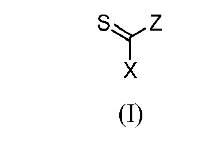 wherein
   X is a substituted or unsubstituted heteroaryl or O-R₁;
   wherein R₁ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, -(C1-C10)(substituted or unsubstituted aryl) and substituted or unsubstituted aryl;
   Z is W-R₂ or NR₃R₄
   wherein W is S or O;
   wherein R₂ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl, substituted or unsubstituted heteroaryl, -(C1-C10)(substituted or unsubstituted aryl or heteroaryl), -(C1-C10)OH, -(CO-C10)C(O)R₅, -(CO-C10)C(O)OR₅, -(C1-C10)OC(O)R₅, -(C1-C10)OC(S)R₅ and -(C1-C10)OR₅;
   wherein R₅ is selected from the group consisting of H, substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl and substituted or unsubstituted heteroaryl;
   wherein R₃ and R₄ independently from each other are selected from the group consisting of H, substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl, substituted or unsubstituted heteroaryl, -(C1-C10)OH, - (C0-C10)C(O)R₅, -(C0-C10)C(O)OR₅, -(C1-C10)OC(O)R₅, -(C1-C10)OC(S)R₅, -(C1-C10)NHC(S)R₅, -(C1-C10)NHC(O)R₅ and -(C1-C10)OR₅; or
   R₃ and R₄ are joined together to form a substituted or unsubstituted heteroaryl; or
   NR₃R₄ form a structure of the following formula (II): 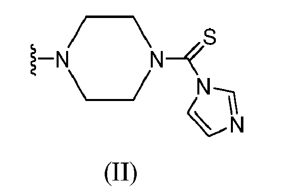 or a pharmaceutically acceptable salt thereof.
3. Pharmaceutical composition according to 2, wherein said COS releasing compound is a compound of formula (I) wherein X is a substituted or unsubstituted heteroaryl selected from the group consisting of imidazolyl, triazolyl and benzimidazolyl.
4. Pharmaceutical composition according to 2 or 3, wherein said COS releasing compound is a compound of formula (I) wherein Z is W-R₂ with R₂ being an unsubstituted C1-C10 alkyl, preferably selected from the group consisting of methyl, ethyl, propyl, isopropyl and butyl.
5. Pharmaceutical composition according to 2 or 3, wherein said COS releasing compound is a compound of formula (I) wherein Z is NR₃R₄ with both R₃ and R₄ being an unsubstituted C1-C10 alkyl, preferably selected from the group consisting of ethyl, propyl, butyl, isobutyl, hexyl and octyl.
6. Pharmaceutical composition according to 2, wherein said COS releasing compound is a compound of formula (I) wherein X is O-R₁ and Z is W-R₂ with W being O resulting in a compound of formula (III): 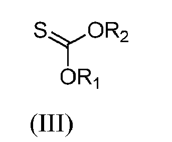 wherein R₁ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, -(C1-C10)(substituted or unsubstituted aryl) and substituted or unsubstituted aryl;
   wherein R₂ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl, substituted or unsubstituted heteroaryl, -(C1-C10)(substituted or unsubstituted aryl or heteroaryl), -(C1-C10)OH, -(C0-C10)C(O)R₅, -(C0-C10)C(O)OR₅, -(C1-C10)OC(O)R₅, -(C1-C10)OC(S)R₅ and -(C1-C10)OR₅;
   wherein R₅ is selected from the group consisting of H, substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl and substituted or unsubstituted heteroaryl;
   or a pharmaceutically acceptable salt thereof.
7. Pharmaceutical composition according to any of 1 to 6, wherein said composition comprises said COS releasing compound as the only pharmaceutically active agent.
8. Pharmaceutical composition according to any of 1 to 7 for the use in the treatment and/or prevention of a cardiovascular disease.
9. Pharmaceutical composition according to 8, wherein said disease is selected from the group of diseases comprising infarction, preferably myocardial infarction; ischemia- reperfusion injuries; protection of organs during surgery, preferably heart surgery or a transplantation surgery; misregulated blood pressure, preferably increased blood pressure; circulatory disorders; cramps and motility-problems of the gastrointestinal tract; irritable bowel syndrome; cramps and dyskinesia of the biliary and urinary tract; cholecystopathia; dysmenorrhoidal problems; cramps at the female genital organs as well as spasms of the muscular soft tissues during delivery (tokolyse); pylorospasms of a baby; spasms during physicals, such as during gastroduodenal-endoscopy or during X-ray; arterial hypertension; pulmonary hypertension; therapy of the hypertension-crisis; coronary heart diseases, such as chronic stable angina pectoris or vasospastic angina; chronic insufficiency of the heart; arterial occlusions, such as Claudicatio intermittens; ischemic neurological deficiencies due to cerebral vasospasms following aneurysmatic subarachnoidal-bleeding; deficiencies of the brain during ageing; bronchospasms during asthma bronchiale; spastic bronchitis; Status Asthmaticus; Morbus Raynaud and vascular-caused cramps of the calves and the toes; and increased intraocular pressure (glaucoma).
10. Method of preparing a pharmaceutical composition comprising a COS releasing compound comprising at least the steps of:
   a) Providing a COS releasing compound;
   b) Mixing said compound with at least one pharmaceutically acceptable excipient.
11. Method according to 10, wherein said COS releasing compound is a compound of formula (I): 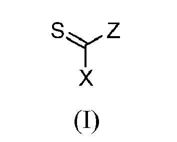 wherein
   X is a substituted or unsubstituted heteroaryl or O-R₁;
   wherein R₁ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, -(C1-C10)(substituted or unsubstituted aryl) and substituted or unsubstituted aryl;
   Z is W-R₂ or NR₃R₄
   wherein W is S or O;
   wherein R₂ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl, substituted or unsubstituted heteroaryl, -(C1-C10)(substituted or unsubstituted aryl or heteroaryl), -(C1-C10)OH, -(C0-C10)C(O)R₅, -(C0-C10)C(O)OR₅, -(C1-C10)OC(O)R₅, -(C1-C10)OC(S)R₅ and -(C1-C10)OR₅;
   wherein R₅ is selected from the group consisting of H, substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl and substituted or unsubstituted heteroaryl;
   wherein R₃ and R₄ independently from each other are selected from the group consisting of H, substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl, substituted or unsubstituted heteroaryl, -(C1-C10)OH, - (C0-C10)C(O)R₅, -(C0-C10)C(O)OR₅, -(C1-C10)OC(O)R₅, -(C1-C10)OC(S)R₅, -(C1-C10)NHC(S)R₅, -(C1-C10)NHC(O)R₅ and -(C1-C10)OR₅; or
   R₃ and R₄ are joined together to form a substituted or unsubstituted heteroaryl; or
   NR₃R₄ form a structure of the following formula (II): 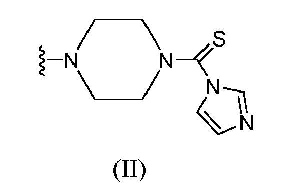 or a pharmaceutically acceptable salt thereof.
12. Method according to 10 or 11, wherein said COS releasing compound is a compound of formula (I) wherein X is O-R₁ and Z is W-R₂ with W being O resulting in a compound of formula (III): 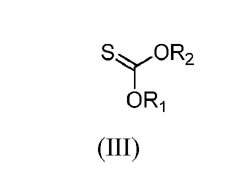 wherein R₁ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, -(C1-C10)(substituted or unsubstituted aryl) and substituted or unsubstituted aryl;
   wherein R₂ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl, substituted or unsubstituted heteroaryl, -(C1-C10)(substituted or unsubstituted aryl or heteroaryl), -(C1-C10)OH, -(C0-C10)C(O)R₅, -(C0-C10)C(O)OR₅, -(C1-C10)OC(O)R₅, -(C1-C10)OC(S)R₅ and -(C1-C10)OR₅;
   wherein R₅ is selected from the group consisting of H, substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl and substituted or unsubstituted heteroaryl;
   or a pharmaceutically acceptable salt thereof.
13. Use of COS or a COS releasing compound as a vasodilator.
14. Use according to 13, wherein said COS releasing compound is a compound of formula (I): 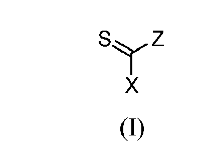 wherein
   X is a substituted or unsubstituted heteroaryl or O-R₁;
   wherein R₁ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, -(C1-C10)(substituted or unsubstituted aryl) and substituted or unsubstituted aryl;
   Z is W-R₂ or NR₃R₄
   wherein W is S or O;
   wherein R₂ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl, substituted or unsubstituted heteroaryl, -(C1-C10)(substituted or unsubstituted aryl or heteroaryl), -(C1-C10)OH, -(C0-C10)C(O)R₅, -(C0-C10)C(O)OR₅, -(C1-C10)OC(O)R₅, -(C1-C10)OC(S)R₅ and -(C1-C10)OR₅;
   wherein R₅ is selected from the group consisting of H, substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl and substituted or unsubstituted heteroaryl;
   wherein R₃ and R₄ independently from each other are selected from the group consisting of H, substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl, substituted or unsubstituted heteroaryl, -(C1-C10)OH, - (C0-C10)C(O)R₅, -(C0-C10)C(O)OR₅, -(C1-C10)OC(O)R₅, -(C1-C10)OC(S)R₅, -(C1-C10)NHC(S)R₅, -(C1-C10)NHC(O)R₅ and -(C1-C10)OR₅; or
   R₃ and R₄ are joined together to form a substituted or unsubstituted heteroaryl; or
   NR₃R₄ form a structure of the following formula (II): 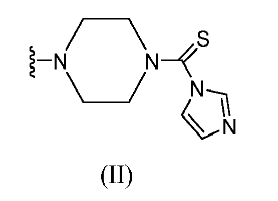 or a pharmaceutically acceptable salt thereof.
15. Use according to 13 or 14, wherein said COS releasing compound is a compound of formula (I) wherein X is O-R₁ and Z is W-R₂ with W being O resulting in a compound of formula (III): 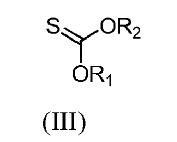 wherein R₁ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, -(C1-C10)(substituted or unsubstituted aryl) and substituted or unsubstituted aryl;
   wherein R₂ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl, substituted or unsubstituted heteroaryl, -(C1-C10)(substituted or unsubstituted aryl or heteroaryl), -(C1-C10)OH, -(C0-C10)C(O)R₅, -(C0-C10)C(O)OR₅, -(C1-C10)OC(O)R₅, -(C1-C10)OC(S)R₅ and -(C1-C10)OR₅;
   wherein R₅ is selected from the group consisting of H, substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl and substituted or unsubstituted heteroaryl;
   or a pharmaceutically acceptable salt thereof.

## Claims

1. A pharmaceutical composition comprising a COS releasing compound of formula (I) 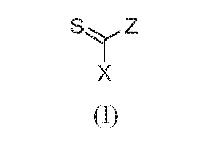 wherein
X is a substituted or unsubstituted heteroaryl or O-R₁;
wherein R₁ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, -(C1-C10)(substituted or unsubstituted aryl) and substituted or unsubstituted aryl;
Z is W-R₂ or NR₃R₄
wherein W is S or O;
wherein R₂ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl, substituted or unsubstituted heteroaryl, -(C1-C10)(substituted or unsubstituted aryl or heteroaryl), -(C1-C10)OH, -(C0-C10)C(O)R₅, -(C0-C10)C(O)OR₅, -(Cl-C10)OC(O)R₅, -(C1-C10)OC(S)R₅ and -(C1-C10)OR₅;
wherein R₅ is selected from the group consisting of H, substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl and substituted or unsubstituted heteroaryl;
wherein R₃ and R₄ independently from each other are selected from the group consisting ofH, substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl, substituted or unsubstituted heteroaryl, -(C1-C10)OH, - (C0-C10)C(O)R₅, -(C0-C10)C(O)OR₅, -(C1-C10)OC(O)R₅, -(C1-C10)OC(S)R₅, -(C1-C10)NHC(S)R₅, -(C1-C10)NHC(O)R₅ and -(C1-C10)OR₅; or
R₃ and R₄ are joined together to form a substituted or unsubstituted heteroaryl; or
NR₃R₄ form a structure of the following formula (II): 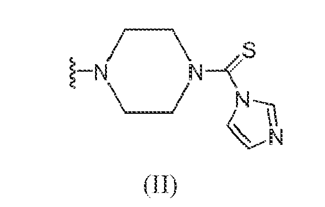 or a pharmaceutically acceptable salt thereof;
for the use in the treatment and/or prevention of a cardiovascular disease selected from the group of diseases comprising infarction, preferably myocardial infarction; ischemia- reperfusion injuries; protection of organs during surgery, preferably heart surgery or a transplantation surgery; misregulated blood pressure, preferably increased blood pressure; circulatory disorders; cramps and motility-problems of the gastrointestinal tract; irritable bowel syndrome; cramps and dyskinesia of the biliary and urinary tract; cholecystopathia; dysmenorrhoidal problems; cramps at the female genital organs as well as spasms of the muscular soft tissues during delivery (tokolyse); pylorospasms of a baby; spasms during physicals, such as during gastroduodenal-endoscopy or during X-ray; arterial hypertension; pulmonary hypertension; therapy of the hypertension-crisis; coronary heart diseases, such as chronic stable angina pectoris or vasospastic angina; chronic insufficiency of the heart; arterial occlusions, such as Claudicatio intermittens; ischemic neurological deficiencies due to cerebral vasospasms following aneurysmatic subarachnoidal-bleeding; deficiencies of the brain during ageing; bronchospasms during asthma bronchiale; spastic bronchitis; Status Asthmaticus; Morbus Raynaud and vascular-caused cramps of the calves and the toes; and increased intraocular pressure (glaucoma).

2. Pharmaceutical composition according to claim 1, wherein said COS releasing compound is a compound of formula (1) wherein X is a substituted or unsubstituted heteroaryl selected from the group consisting of imidazolyl, triazolyl and benzimidazolyl.

3. Pharmaceutical composition according to claim 1 or 2, wherein said COS releasing compound is a compound of formula (I) wherein Z is W-R₂ with R₂ being an unsubstituted C1-C10 alkyl, preferably selected from the group consisting of methyl, ethyl, propyl, isopropyl and butyl.

4. Pharmaceutical composition according to claims 1 or 2, wherein said COS releasing compound is a compound of formula (1) wherein Z is NR₃R₄ with both R₃ and R₄ being an unsubstituted C1-C10 alkyl, preferably selected from the group consisting of ethyl, propyl, butyl, isobutyl, hexyl and octyl.

5. Pharmaceutical composition according to claim 1, wherein said COS releasing compound is a compound of formula (I) wherein X is O-R₁ and Z is W-R₂ with W being O resulting in a compound of formula (III): 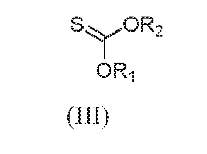 wherein R₁ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, -(C1-C10)(substituted or unsubstituted aryl) and substituted or unsubstituted aryl;
wherein R₂ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl, substituted or unsubstituted heteroaryl, -(C1-C10)(substituted or unsubstituted aryl or heteroaryl), -(C1-C10)OH, -(C0-C10)C(O)R₅, -(C0-C10)C(O)OR₅, -(C1-C10)OC(O)R₅, -(C1-C10)OC(S)R₅ and -(C1-C10)OR₅;
wherein R₅ is selected from the group consisting of H, substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl and substituted or unsubstituted heteroaryl;
or a pharmaceutically acceptable salt thereof.

6. A pharmaceutical composition comprising a COS releasing compound.

7. Pharmaceutical composition according to claim 6, wherein said COS releasing compound is a compound of formula (I): 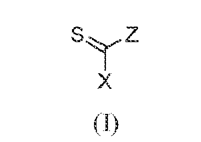 wherein
X is a substituted or unsubstituted heteroaryl or O-R₁;
wherein R₁ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, -(C1-C10)(substituted or unsubstituted aryl) and substituted or unsubstituted aryl;
Z is W-R₂ or NR₃R₄
wherein W is S or O;
wherein R₂ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl, substituted or unsubstituted heteroaryl, -(C1-C10)(substituted or unsubstituted aryl or heteroaryl), -(C1-C10)OH, -(C0-C10)C(O)R₅, -(C0-C10)C(O)OR₅, -(C1-C10)OC(O)R₅, -(C1-C10)OC(S)R₅ and -(C1-C10)OR₅;
wherein R₅ is selected from the group consisting of H, substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl and substituted or unsubstituted heteroaryl;
wherein R₃ and R₄ independently from each other are selected from the group consisting ofH, substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl, substituted or unsubstituted heteroaryl, -(C1-C10)OH, - (C0-C10)C(O)R₅, -(C0-C10)C(O)OR₅, -(C1-C10)OC(O)R₅, -(C1-C10)OC(S)R₅, -(C1-C10)NHC(S)R₅, -(C1-C10)NHC(O)R₅ and -(C1-C10)OR₅; or
R₃ and R₄ are joined together to form a substituted or unsubstituted heteroaryl; or
NR₃R₄ form a structure of the following formula (II): 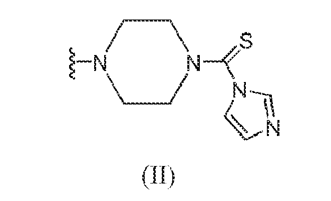 or a pharmaceutically acceptable salt thereof.

8. Pharmaceutical composition according to claim 6 or 7, wherein said COS releasing compound is a compound of formula (I) wherein X is O-R₁ and Z is W-R₂ with W being O resulting in a compound of formula (III): 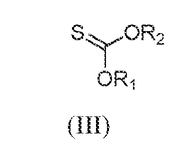 wherein R₁ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, -(C1-C10)(substituted or unsubstituted aryl) and substituted or unsubstituted aryl;
wherein R₂ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl, substituted or unsubstituted heteroaryl, -(C1-C10)(substituted or unsubstituted aryl or heteroaryl), -(C1-C10)OH, -(C0-C10)C(O)R₅, -(C0-C10)C(O)OR₅, -(C1-C10)OC(O)R₅, -(C1-C10)OC(S)R₅ and -(C1-C10)OR₅;
wherein R₅ is selected from the group consisting of H, substituted or unsubstituted C1-C10 alkyl, substituted or unsubstituted C3-C8 cycloalkyl,
substituted or unsubstituted C2-C10 alkenyl, substituted or unsubstituted C4-C8 cycloalkenyl, substituted or unsubstituted aryl and substituted or unsubstituted heteroaryl;
or a pharmaceutically acceptable salt thereof.

9. Pharmaceutical composition according to any of claims 1 to 8, wherein said composition comprises said COS releasing compound as the only pharmaceutically active agent.

10. Method of preparing a pharmaceutical composition comprising a COS releasing compound comprising at least the steps of:
a) Providing a COS releasing compound;
b) Mixing said compound with at least one pharmaceutically acceptable excipient.

11. Method according to claim 10, wherein said COS releasing compound is a compound of formula (I): 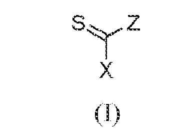 wherein
X is a substituted or unsubstituted heteroaryl or O-R₁;
wherein R₁ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, -(C1-C10)(substituted or unsubstituted aryl) and substituted or unsubstituted aryl;
Z is W-R₂ or NR₃R₄
wherein W is S or O;
wherein R₂ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl, substituted or unsubstituted heteroaryl, -(C1-C10)(substituted or unsubstituted aryl or heteroaryl), -(C1-C10)OH, -(C0-C10)C(O)R₅, -(C0-C10)C(O)OR₅, -(C1-C10)OC(O)R₅, -(C1-C10)OC(S)R₅ and -(C1-C10)OR₅;
wherein R₅ is selected from the group consisting of H, substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl and substituted or unsubstituted heteroaryl;
wherein R₃ and R₄ independently from each other are selected from the group consisting of H, substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl, substituted or unsubstituted heteroaryl, -(C1-C10)OH, - (C0-C10)C(O)R₅, -(C-C10)C(O)OR₅, -(C1-C10)OC(O)R₅, -(C1-C10)OC(S)R₅, -(C1-C10)NHC(S)R₅, -(C1-C10)NHC(O)R₅ and -(C1-C10)OR₅; or
R₃ and R₄ are joined together to form a substituted or unsubstituted heteroaryl; or
NR₃R₄ form a structure of the following formula (II): 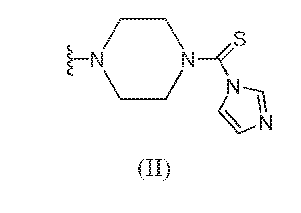 or a pharmaceutically acceptable salt thereof.

12. Method according to claim 10 or 11, wherein said COS releasing compound is a compound of formula (I) wherein X is O-R₁ and Z is W-R₂ with W being O resulting in a compound of formula (III): 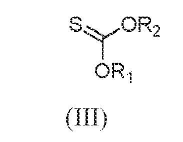 wherein R₁ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, -(C1-C10)(substituted or unsubstituted aryl) and substituted or unsubstituted aryl;
wherein R₂ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl, substituted or unsubstituted heteroaryl, -(C1-C10)(substituted or unsubstituted aryl or heteroaryl), (C1-C10)OH, -(C0-C10)C(O)R₅, -(C0-C10)C(O)OR₅, -(C1-C10)OC(O)R₅, -(C1-C10)OC(S)R₅ and -(C1-C10)OR₅;
wherein R₅ is selected from the group consisting of H, substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl and substituted or unsubstituted heteroaryl;
or a pharmaceutically acceptable salt thereof.

13. Use of COS or a COS releasing compound as a vasodilator.

14. Use according to claim 13, wherein said COS releasing compound is a compound of formula (I): 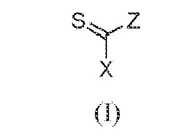 wherein
X is a substituted or unsubstituted heteroaryl or O-R₁;
wherein R₁ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, -(C1-C10)(substituted or unsubstituted aryl) and substituted or unsubstituted aryl;
Z is W-R₂ or NR₃R₄
wherein W is S or O;
wherein R₂ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl, substituted or unsubstituted heteroaryl, -(C1-C10)(substituted or unsubstituted aryl or heteroryl), (C1-C10)OH, -(C0-C10)C(O)R₅, -(C0-C10)C(O)OR₅, -(C1-C10)OC(O)R₅, -(C1-C10)OC(S)R₅ and -(C1-C10)OR₅;
wherein R₅ is selected from the group consisting of H, substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl and substituted or unsubstituted heteroaryl;
wherein R₃ and R₄ independently from each other are selected from the group consisting ofH, substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl, substituted or unsubstituted heteroaryl, -(C1-C10)OH, - (C0-C10)C(O)R₅, -(C0-C10)C(O)OR₅, -(C1-C10)OC(O)R₅, -(C1-C10)OC(S)R₅ , -(C1-C10)NHC(S)R₅, -(C1-C10)NHC(O)R₅ and -(C1-C10)OR₅; or
R₃ and R₄ are joined together to form a substituted or unsubstituted heteroaryl; or
NR₃R₄ form a structure of the following formula (II): 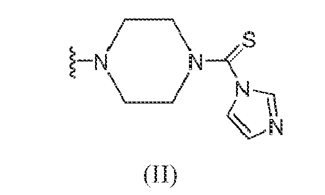 or a pharmaceutically acceptable salt thereof.

15. Use according to claim 13 or 14, wherein said COS releasing compound is a compound of formula (I) wherein X is O-R₁ and Z is W-R₂ with W being O resulting in a compound of formula (III): 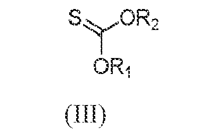 wherein R₁ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, -(C1-C10)(substituted or unsubstituted aryl) and substituted or unsubstituted aryl;
wherein R₂ is selected from the group consisting of substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl, substituted or unsubstituted heteroaryl, (C1-C10)(substituted or unsubstituted aryl or heteroaryl), -(C1-C10)OH, -(C0-C10)C(O)R₅, -(C0-C10)C(O)OR₅, -(C1-C10)OC(O)R₅, -(C1-C10)OC(S)R₅ and -(C1-C10)OR₅;
wherein R₅ is selected from the group consisting of H, substituted or unsubstituted C1-C10 alkyl, substituted or unsubstituted C3-C8 cycloalkyl, substituted or unsubstituted C2-C10 alkenyl, substituted or unsubstituted C4-C8 cycloalkenyl, substituted or unsubstituted aryl and substituted or unsubstituted heteroaryl;
or a pharmaceutically acceptable salt thereof.
